# EUROPEAN PATENT SPECIFICATION

(11) **EP 3 485 036 B1**
(45) Date of publication and mention of the grant of the patent: **17.03.2021**
(21) Application number: 17746010.2
(22) Date of filing: 14.07.2017
(51) Int. Cl.: C12Q 1/68

(54) **NUCLEOTIDE-BASED NANOSWITCH AND METHODS FOR THE DETECTION OF ANTIBODIES AND OTHER ANALYTES**
NUKLEOTIDBASIERTER NANOSCHALTER UND VERFAHREN ZUR DETEKTION VON ANTIKÖRPERN UND ANDEREN ANALYTEN
NANOCOMMUTATEUR À BASE DE NUCLÉOTIDES ET MÉTHODES DE DÉTECTION D'ANTICORPS ET D'AUTRES ANALYTES

(30) Priority: 14.07.2016 IT 201600073964
(43) Date of publication of application: 22.05.2019
(73) Proprietor: Ulisse Biomed S.R.L., 33100 Udine (IT)
(72) Inventor: IPPODRINO, Rudy, 34135 Trieste (IT); MARINI, Bruna, 34136 Trieste (IT); RICCI, Francesco, 00162 Roma (IT); PORCHETTA, Alessandro, 00043 Ciampino (IT)
(74) Representative: Petraz, Gilberto Luigi
(86) International application number: PCT/EP2017/067904
(87) International publication number: WO 2018/011412

(56) References cited:
- WO-A1-2015/118029
- WO-A2-2005/098049

## Description

### FIELD OF THE INVENTION

Embodiments of the present disclosure relate to a nucleotide-based nanoswitch, or probes system, for detecting one or more targets in a sample and methods for the detection of said one or more targets, in particular antibodies, protein antigens and other protein analytes.

### BACKGROUND OF THE INVENTION

Nowadays sensitive assays for the detection of proteins, antibodies or other analytes have several drawbacks mainly due to the fact that most of these methods are multistep, washing-intensive and reagent-intensive processes. For this reason, these methods are slightly suitable for medical diagnostic point-of-care applications. In the following, a brief discussion of the commonly used assays and methods is provided.

### Lateral Flow

Lateral-flow based immunoassays represent the fastest and the cheapest format test for diagnostic point of care application. Some lateral-flow tests can directly work on complex matrices and present a result in matter of few minutes, but is often required sample processing steps before the loading. The result is qualitative, and in one-step format neither a washing step is possible to clean the reaction, nor it is possible to enhance the response by enzyme reaction; the imprecise sample volume reduces precision, moreover given that the total volume used is very limited this might impact on sensitivity. Lateral flow are exploited for individual tests, and not for high-throughput screening.

### ELISA

Currently, Enzyme-linked immunosorbent assay (ELISA) tests are the most diffused on the diagnostic market, these assays are used to measure clinically relevant toxins, antigens, antibodies and chemicals. ELISA are pretty complex tests, they are time consuming and specialized personell are required for their execution.

### Electrochemistry

Capacitive, amperometric or optic biosensor can be assembled in cheaper and faster platform compared to standard ELISA immunoenzymatic tests. They are suitable both for an individual assay and for the analysis of many samples in parallel. They can reach high sensitivity and high sensibility, and still give a quantitative result. Microfluidics can be easily implemented if washes or amplification of the signal are needed. Despite these good features, the majority of electrochemical immunosensors require samples separation or washing steps.

### RIA and SPR

Radioimmunoassay (RIA) and plasmon surface resonance (SPR) are very potent techniques able to measure the presence of analytes with high sensitivity but at the same time they are far from the medical point of care market because are expensive, not user friendly and the handling of toxic reagents is not rare.

### OTHER METHODS

Methods that exploit nucleotide-based probes are promising due to their cost-effectiveness and rapidity: the binding of an analyte as an antibody for example can induce a conformational change of a nucleotidic, stem-loop scaffold, that is conjugated to two binding moiety; due to the conformational change of the scaffold structure, one or more signaling moieties are activated and a signal is triggered. One example is described in WO-A-2012/071344. However, the design of the nucleotide-based beacon nanostructure is strictly dependent on the affinity and the avidity of the antibodies, parameters that serve to stretch and activate the signaling component of the system, but that can strongly variate depending on the patient. Moreover, the steric hindrance of the binding moiety represents a limiting step in the design of the molecular beacon structure.

Document WO-A-2015/118029 (WO'029) a method for the detection of analytes based on a so-called proximity strategy, based on Hybridization Chain Reaction, in particular a proximity-based amplification of the signalling generated by an initiator oligo.

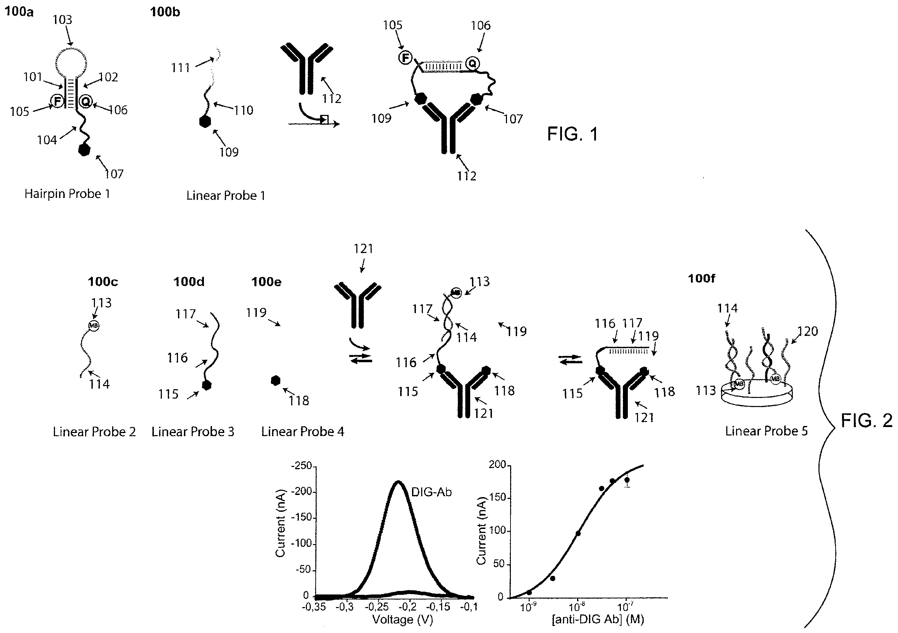

In particular, the general scheme of WO'029 is shown in FIG. 1, where the following components are required:
i) a first DNA metastable hairpin conjugated with an antibody;
ii) a second DNA metastable hairpin conjugated with a different antibody;
iii) a DNA strand called "activation oligo";
iv) a third DNA metastable hairpin conjugated with a fluorophore;
v) a fourth DNA metastable hairpin conjugated with a fluorophore.

The method of WO'029 requires 5 different oligonucleotides in order to measure an antigen. In WO'029 these elements, through the interaction triggered by the target molecule, react with each other giving a measurable signal. It is important to note that the presence of the "activation oligo" appears to be crucial for the strategy of WO'029. Without the activation oligo, the system of WO'029 does not work. Similarly, in WO'029 it is crucial to have 4 different metastable hairpins, each of them conjugated to different tags (antibodies or fluorophores). These aspects make the whole approach of WO'029 quite complex, requiring the addition of several components in order to have a measurable signal compared to our system.

Moreover, the strategy proposed by WO'029 is mainly directed to the detection of analytes exploiting two typologies of binding moieties:
a) an antibody that specifically binds the analyte and coupled to the DNA hairpin;
b) a factor able to interact with the analyte;
c) an antibody that specifically bind the factor interacting with the analyte, and coupled to the DNA hairpin.

This renders the system much more complex, because there are three binding moments that needs to occur in order to trigger the signal: the antibody specific for the analyte needs to bind the analyte, the factor interacting with the analyte needs to interact with the analyte, and finally the antibody specific for the interacting factor needs to bind the factor.

Moreover, it shall be underlined that WO'0291 specifically requires a deprotecting unit (activator oligo) which is essential to the whole process. This means that the presence of the activator oligo is a limiting step to start the sensing process, making the whole method of WO'029 more complex.

The signalling in WO'029, see FIG: 1, is based on the binding of the target molecule and of an activation oligo to the metastable hairpins. The activator oligo is a crucial part for the sensing strategy of WO'029 and may represent the weak point for the entire process described therein. As shown in WO'029, the use of a mismatch activator oligo gives a very low signal when compared to the full match activator oligo. This means that any factor affecting the binding of the activation oligo to the metastable hairpin might affect the overall measurement.

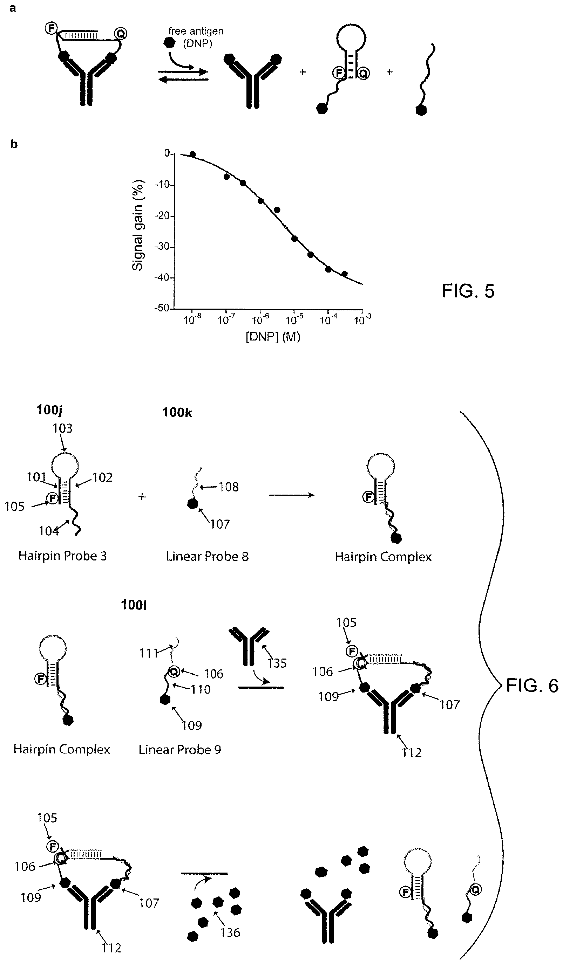

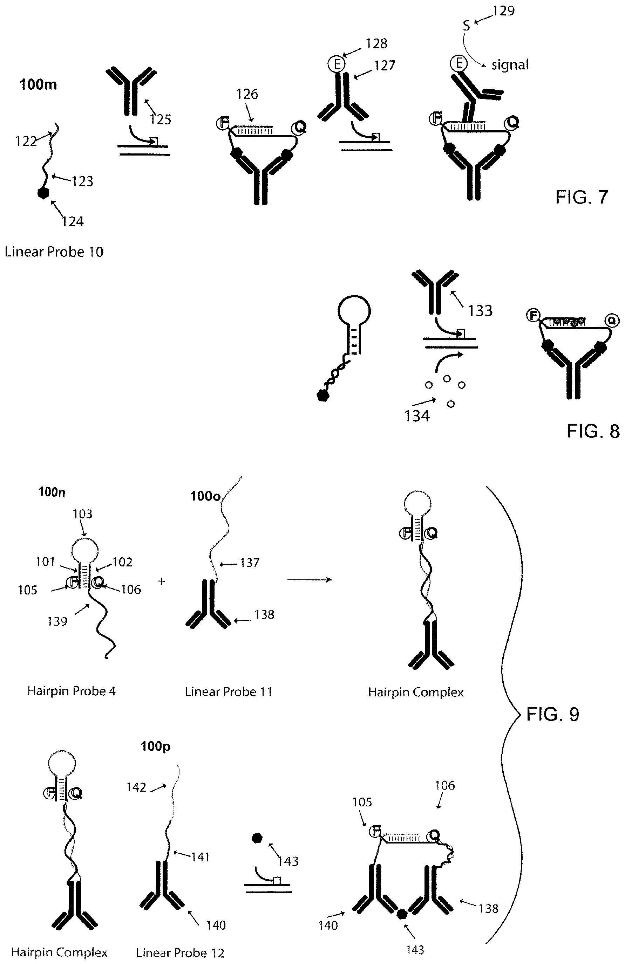

Moreover, it shall be underlined that in WO'029 "control experiments" are shown where the activator oligo is not inserted in the absence of the specific target. All the control experiments show the presence of the protein but the lack of the "activator oligo" (see for example FIG. 5B of WO'029). This does not represent a proper control, because it is not possible to quantify the presence of background signal in the absence of the target. The results that include a correctly performed control (FIG. 8B of WO'029) support this observation. They clearly show that in the presence of a non specific target (primary rabbit antibody) and the inititator oligo (right panel), there is a strong background signal that makes hard to distinguish the main signal (mouse antibody, FIG. 8A, right panel). This means that the HCR is partially activated by the sole presence of the "activator oligo". This can be clearly observed in the FIG. 14B of WO'029 (Surface Plasmon Resonance results) that shows the reaction between the proximity hairpin 1, proximity hairpin 2 and initiator oligo. This graph clearly shows that the reaction occurs even in the absence of the target antigen.

Again, WO'029 proposes the possible use of fluorophore/quencher pair associated with the metastable hairpin. However, it should be noted that, as the strategy of WO'029 is designed, the platform would lead to an high background signal because the metastable hairpin can be activated also by only the initiator oligo and without the presence of the target antigen. No results are reported with a fluorophore/quencher pair in WO'029.

Moreover, WO'029 has a further inherent limitation, namely a long incubation time to obtain a measurable signal is required. WO'029 reports that the signal peaks occur after 2 hours. Indeed the system presented in WO'029 is based on an Hybridization Chain Reaction which requires a lot of time to occur and possibly multiple washes are needed.

It shall be further noted that WO'029 requires 4 different DNA oligos in order to work properly. This feature makes higher the cost of the system of WO'029.

Document WO-A-2005/098049 (WO'049) discloses metastable signalling probes with hairpin structures, which are commonly quoted in a lot of nucleic-acid beacon related patents. The probes interact only when the target initiator is present which triggers the conformational change that lead to a signaling cascade. However, document WO'049 is suitable only for DNA targets detection. WO'049 does not disclose any method for the detection of non-nucleotide targets and, moreover, it is not based on any proximity effect.

Document WO-A-2007/15242 (WO'242) is also known, disclosing binary probes for fluorescent analysis of nucleic acids. The binary probes interact and assemble in the presence of the target. The assembly creates a binding site for a beacon such that it linearizes generating a signal. However, WO'242 refers to the detection of nucleic acids targets and it is not able to detect protein analytes. In particular, WO'242 discloses a system where two probes are assembled after binding with a target. The assembled probes can thereby bind to a molecular beacon and linearize it. WO0242 shows that the target makes possible the formation of a complex between two probes and this complex can then bind to the molecular beacon.

There is therefore a need to improve the detection of one or more targets in a complex sample and methods for the detection of said one or more targets which overcome at least one of the drawbacks in the art.

The Applicant has devised, tested and embodied the present invention, including a nucleotide-based nanoswitch, or probes systems and methods, based on the increase of local concentration of the probes, to overcome the shortcomings of the state of the art and to obtain these and other purposes and advantages.

Various limitations and disadvantages of conventional solutions and technologies will become apparent to one of skill in the art after reviewing the remainder of the present application with reference to the drawings and description of the embodiments which follow, though it should be understood that this description of the related art section is not intended to serve as an admission that the described subject matter is prior art.

### SUMMARY OF THE INVENTION

The present invention is set forth and characterized in the independent claims, while the dependent claims describe other characteristics of the invention or variants to the main inventive idea.

According to the invention, nucleotide-based nanoswitch, or probes system for detecting one or more targets in a sample is provided. In one embodiment, the system comprises a set of two or more oligonucleotide probes configured to provide a target binding-induced structural change of one or more of the oligonucleotide probes by a proximity-based hybridization reaction triggered by an increase in local concentration of the two or more oligonucleotide probes, wherein, upon contact of said oligonucleotide probes with the one or more targets, hybridization between hybridization sequences of said probes occurs, thereby producing a detectable change in a signal from one of the probes when the one or more of the oligonucleotide probes are contacted by the one or more targets.

According to the invention, said probes comprise:
a first probe containing a target binding moiety and a hybridization sequence;
a second probe containing a second target binding moiety and at least a hybridization sequence configured to form a duplex with the hybridization sequence of the first probe, said second probe further comprising a first signaling moiety and a second signaling moiety, wherein the first signaling moiety comprises an electrochemical reporter and the second signaling moiety comprises an electrode.

According to further embodiments, methods using nucleotide-based nanoswitch, or probes systems, according to the present disclosure, for detecting one or more targets in a sample, are also provided.

These and other features, aspects and advantages of the present disclosure will become better understood with reference to the following description, the drawings and appended claims. The drawings, which are incorporated in and constitute a part of this specification, illustrate embodiments of the present subject matter and, together with the description, serve to explain the principles of the disclosure.

According to the invention, combinable with all embodiments described herein, said one or more targets are protein analytes, in particular an antibody or a protein antigen.

The various aspects and features described in the present disclosure can be applied, individually, wherever possible. These individual aspects, for instance the aspects and features described in the attached dependent claims, can be made subject of divisional patent applications.

It is noted that anything found to be already known during the patenting process is understood not to be claimed and to be the subject of a disclaimer.

### BRIEF DESCRIPTION OF THE DRAWINGS

These and other characteristics of the present invention will become apparent from the following description of embodiments, given as a non-restrictive example with reference to the attached drawings wherein:
- FIG. 1 shows the set of two oligonucleotide probes configured to produce a detectable signal upon target binding, according to embodiments of the present disclosure;
- FIG. 2 (upper panels) shows oligonucleotide probes that uses an electrochemical reporter (e.g., methylene blue) and an electrode as the two signaling moieties, according to embodiments of the present disclosure. FIG. 2 (lower panels) shows graphs of square wave voltammograms and current signal vs target concentration for anti-Dig antibody probes, according to embodiments of the present disclosure;

- FIG. 3 shows the set of at least three oligonucleotide probes configured to produce a detectable signal upon target binding, according to embodiments of the present disclosure;
- FIG. 4 shows the set of oligonucleotide probes where the target binding can be inhibited by a second target, being this inversely proportional to the detectable signal generated from the first target, according to embodiments of the present disclosure;
- FIG. 5 shows the embodiment wherein the probes are used in a competitive assay to detect an antigen, that is identical to the target binding moieties present on the probes, according to embodiments of the present disclosure;
- FIG. 6 shows the embodiment wherein the probes are used in a competitive assay to detect an antigen, that is identical to the target binding moieties present on the probes, and the signaling moieties are localized on different probes, according to embodiments of the present disclosure;
- FIG. 7 shows oligonucleotide probes that is constituted partially or totally by an RNA sequence (depicted in gray), according to embodiments of the present disclosure;
- FIG. 8 shows the usage of intercalating dye that binds the hybridized sequences of the two probes, and it produces a fluorescent signal when the target is present, according to embodiments of the present disclosure;
- FIG. 9 shows the embodiment wherein the target is an antigen of interest, and the target binding moieties are constituted by two molecules able to specifically bind the antigen in two different epitopes (e.g. an antibody, or a single chain variable fragment, or a Fab, or an affybody, or an aptabody), according to embodiments of the present disclosure.
- FIG. 10(a) shows a set of oligonucleotide probes configured to produce a detectable fluorescent signal upon target binding, according to embodiments of the present disclosure. The fluorescent signaling moieties are FAM-6 for the fluorophore and BHQ-1 for the quencher;
- FIG. 10(b) shows graphs of signal vs. target concentration for anti-Dig antibody and anti-DNP antibody probes according to embodiments of the present disclosure;
- FIG. 10(c) shows graphs of signal vs. time for anti-Dig antibody and anti-DNP antibody probes according to embodiments of the present disclosure;
- FIG. 11 shows graph of signal vs. target concentration for the anti-DNP antibody probes detected in serum sample according to embodiments of the present disclosure;
- FIG. 12 shows embodiments wherein a modular system of probes is provided to detect a target using two scaffold probes and a single linear probe coupled with target binding moiety.

### DETAILED DESCRIPTION OF THE EMBODIMENTS

Reference will now be made in detail to the various embodiments of the invention, one or more examples of which are illustrated in the figures. Within the following description of the drawings, the same reference numbers refer to the same components. Generally, only the differences with respect to individual embodiments are described. Each example is provided by way of explanation of the invention and is not meant as a limitation of the invention. For example, features illustrated or described as part of one embodiment can be used on or in conjunction with other embodiments to yield yet a further embodiment. It is intended that the present invention includes such modifications and variations.

Embodiments described herein refer to a nucleotide-based nanoswitch, or probes system, for detecting one or more targets in a sample and methods for the detection of said one or more targets.

According to embodiments, combinable with all embodiments described herein, said one or more targets are protein analytes, in particular an antibody or a protein antigen.

In particular, according to the present disclosure, nucleotide-based nanoswitch, or probes system, and methods are provided for the detection of said one or more targets that use target binding-induced structural change of an oligonucleotide probe by a proximity-based hybridization reaction triggered by an increase in local concentration, wherein, upon contact of said oligonucleotide probes with the one or more targets, hybridization between hybridization sequences of said probes occurs.

Accordingly, in some embodiments, a system for detecting one or more targets in a sample is provided. The system may include a set of oligonucleotide probes configured to produce a detectable signal when contacted by the one or more targets.

In some embodiments, the probes use target binding-induced structural changes to detect the presence of the target in the sample by utilizing binding-induced increase of local concentration of two target binding moieties as a signaling mechanism.

Accordingly, the system comprises a set of two or more oligonucleotide probes configured to provide a target binding-induced structural change of one or more of the oligonucleotide probes by a proximity-based hybridization reaction triggered by an increase in local concentration of the two or more oligonucleotide probes wherein, upon contact of said oligonucleotide probes with the one or more targets, hybridization between hybridization sequences of said probes occurs, thereby producing a detectable change in a signal from one of the probes when the one or more of the oligonucleotide probes are contacted by the one or more targets.

Advantageously, the present disclosure is based on quantitative antibody, or in general protein analyte or antigen, detection in bodily fluid (e.g. crude samples), that, in some embodiments, can be carried out through only two probes, contrary to WO'029 that uses 5 different oligonucleotides.

Advantageously, moreover, the embodiments according to the present disclosure do not show any significant background signalling in the absence of the specific target. The features of the claimed invention make it more sensitive and quantitative compared to WO'029 because they act through reducing the background signal.

Moreover, contrary to WO'029, the embodiments described herein are based on a proximity-based hybridization reaction.

According to embodiments which are described using FIG. 1, the probes comprise:
a first probe containing a first target binding moiety, two self-hybridization sequences and a hybridization sequence (Hairpin Probe 1);
a second probe containing a target binding moiety and a hybridization sequence (Linear Probe 1);
wherein the two self-hybridization sequences of the first probe have two self-complementary ends configured to create a stem-loop configuration and the hybridization sequence of the first probe is configured to be complementary to the hybridization sequence of the second probe to form a duplex with the hybridization sequence of the second probe, such that said duplex formation is inhibited in the absence of the target in a concentration-dependent fashion;
said first probe further comprising a first signaling moiety and a second signaling moiety configured such that the position of the first signaling moiety is in proximity of the second signaling moiety in absence of target and in the presence of the stem-loop configuration;
wherein upon binding of the one or more targets to both the first target binding moiety of the first probe and the second target binding moiety of the second probe, an increase in local concentration of the first probe and second probe is caused, the increase in local concentration of the first probe and second probe allowing the hybridization between the two hybridization sequences to form the duplex between the sequence of the first probe and the sequence of the second probe, causing thereby the opening of the stem-loop configuration thus changing the distance between the first signaling moiety and the second signaling moiety in the first probe and producing a detectable change in a signal from the first and second signaling moieties of the second probe.

Advantageously, in the embodiments described using FIG. 1, combinable with all embodiments described herein, as a minimum requirement only two components can be involved, namely:
i) a DNA metastable hairpin (stem-loop) conjugated with an antigen (recognition element) and with a fluorophore/quencher pair;
ii) a DNA "input" strand conjugated with the same antigen (recognition element).

Advantageously, in other words, the present disclosure provides a molecular beacon (i) and an input strand (ii) that are brought together after binding to a target that allows their hybridization. Advantageously, only the target makes possible the binding between the molecular beacon and the input strand.

The binding of the two recognition elements to a single target (for example an antibody in this case) allows the hybridization between the stem-loop and the "input" strand, giving a measurable signal increase. The embodiments according to the present disclosure, contrary to prior art WO'029, do not require any "activation oligo" and only 1 metastable hairpin is involved (instead of 4 hairpins as in WO'029). This aspect guarantees robustness, higher efficiency and sensitivity of the sensor used in the embodiments described herein. The simplicity of the embodiments according to the present disclosure positively correlates with its robustness and efficiency. Advantageously, the embodiments according to the present disclosure are based on the thermodynamic optimization of the metastable hairpin/input strand pair, which should not show a high affinity in the absence of the target molecule. Advantageously, in the claimed invention, the use of only a single metastable hairpin makes the optimization of the system easy and quick compared to a multiple metastable hairpin system.

Advantageously, the embodiments according to the present disclosure are direct and quick, in particular they work directly because the only presence of analyte is able to trigger the signal, without involving other chemical reactions. Indeed, in the embodiments described herein, the signal peaks of are measurable a very short time, for instance in about 3 minutes. This makes the claimed invention suitable for the developing of point of care self-testing systems, and for clinical applications.

Moreover, the use of only two probes makes the cost of the detection system much more cheaper when compared, for instance, with WO'029.

In further embodiments such as those described using FIG. 2, the probes comprise:
a first probe containing a target binding moiety and a hybridization sequence;
a second probe containing a second target binding moiety and a first and a second hybridization sequences (Linear Probe 3),
wherein the first and second hybridization sequences are configured to form a duplex with the hybridization sequence of the first probe;
a third probe (Linear Probe 2) containing a signaling moiety and a hybridization sequence configured to form a duplex with the first hybridization sequence of the second probe,
wherein the duplex formation between the hybridization sequence of the first probe and the first and second hybridization sequences of the second probe is inhibited in the absence of the target;
wherein upon binding of the one or more targets to both the first target binding moiety of the first probe and the second target binding moiety of the second probe an increase in local concentration of the first probe and second probe is caused, the increase in local concentration of the first probe and second probe allowing the formation of the duplex between the hybridization sequence of the first probe and the two hybridization sequences of the second probe to form the duplex between the sequence of the first probe and the sequence of the second probe causing thereby the release of the third probe and producing a detectable change in a signal from the first and second signaling moieties of the second probe.

According to yet further embodiments, the first target binding moiety and the second target binding moiety can be bound directly or indirectly to the two probes.

In embodiments in which the first target binding moiety and the second target binding moiety are bound directly to the two probes, at least one of the first target binding moiety and the second target binding moiety is bound to the two probes through a linker moiety.

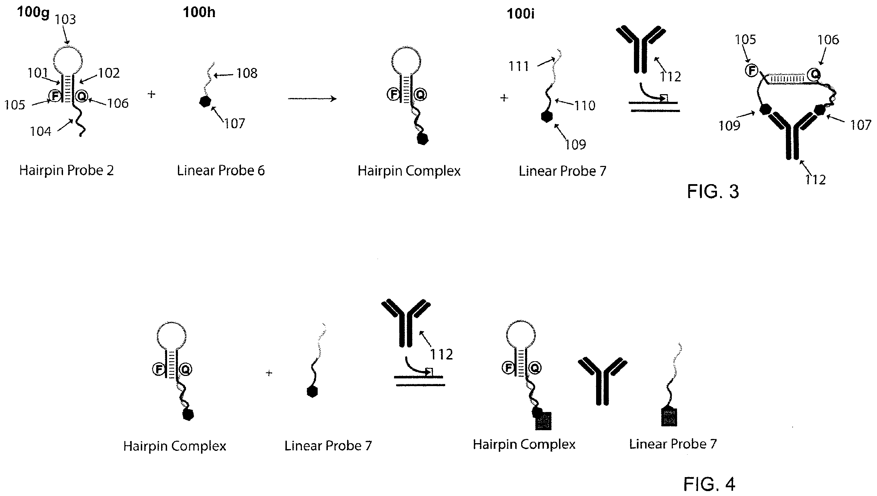

In yet further embodiments such as those described using FIG. 3 in which the first target binding moiety and the second target binding moiety are bound indirectly to the two probes, the set of probes further comprises an additional probe (Linear Probe 6) that contains a target binding moiety and a third hybridization sequence and wherein the first probe (Hairpin Probe 2) does not contain the target binding moiety but also contains an additional hybridization sequence that is configured to bound to the third hybridization sequence of the third probe. Accordingly, in these embodiments, the target binding moiety may be bound to the third hybridization sequence complementary to a tail sequence of the first probe so that the hybridization between the Hairpin Probe 2 and the Linear Probe 6 produces a complex that contains a target binding moiety and the two signaling moieties (Hairpin Complex).

In embodiments, the first target binding moiety and the second target binding moiety comprise antigens, and the target comprises an antibody specific for the antigens.

In further embodiments, the first target binding moiety and the second target binding moiety comprise polypeptides that specifically bind to a macromolecule, and wherein the target comprises the macromolecule.

In yet further embodiments, the first target binding moiety and the second target binding moiety comprise aptamers that specifically bind to a macromolecule, and wherein the target comprises the macromolecule.

In still further embodiments, the first target binding moiety and the second target binding moiety comprise DNA or RNA sequences that specifically bind to a macromolecule, and wherein the target comprises the macromolecule.

According to further embodiments, the first signaling moiety comprises a fluorophore and the second signaling moiety comprises a quencher.

According to yet further embodiments the first signaling moiety comprises a first fluorophore and the second signaling moiety comprises a second fluorophore.

According to still further embodiments first signaling moiety comprises a nanoparticle and the second signaling moiety comprises a quencher.

In further embodiments, the first signaling moiety comprises a first nanoparticle and the second signaling moiety comprises a second nanoparticle.

According to the invention, the first signaling moiety comprises an electrochemical reporter and the second signaling moiety comprises an electrode. Accordingly, the probe may be immobilized on the surface of the electrode.

In still further embodiments, the first signaling moiety comprises a macromolecule having a catalytic activity and the second signaling moiety comprises an inhibitor or an activator of the catalytic activity.

In other embodiments, multiple signaling moieties are bound to the probes, that cooperatively emit a signal, if target is present.

In embodiments, the system may include an array of probes.

According to further embodiments, a method of detecting a target in a sample is provided. In one embodiment, the method comprises:
contacting a set of oligonucleotide probes according to embodiments described herein with the sample, whereby the target selectively binds to both the first target binding moiety and the second target binding moiety to form a complex with the set of probes of embodiments described herein and the target; and detecting the presence or absence of the complex with the set of probes of embodiments described herein and the target.

In embodiments, the sample may comprise a complex sample or whole blood, mucus, urine, blood plasma, saliva, sweat, stools extracts, tissue biopsies, sebaceous secretions, liquid phase of cell and tissue extracts.

According to yet further embodiments, a method of detecting a second target in a sample is provided. In one embodiment described using FIG. 4, the method comprises:
contacting a set of oligonucleotide probes according to embodiments described herein with the sample, whereby the target selectively binds to both the first target binding sequence and the second target binding sequence to form a complex with the set of probes of to embodiments described herein; contacting the first target with a second target, whereby the second target selectively binds the target and inhibits formation of the complex with the set of probes of to embodiments described herein and the target; and detecting the presence or absence of the complex with the set of probes of embodiments described herein and the target.

In possible embodiments, after forming the complex with the set of probes of embodiments described, the target releases the probe containing the signaling moiety, further wherein after inhibiting formation of the complex with the set of probes of embodiments described and the target, the second target releases the probe containing the signaling moiety.

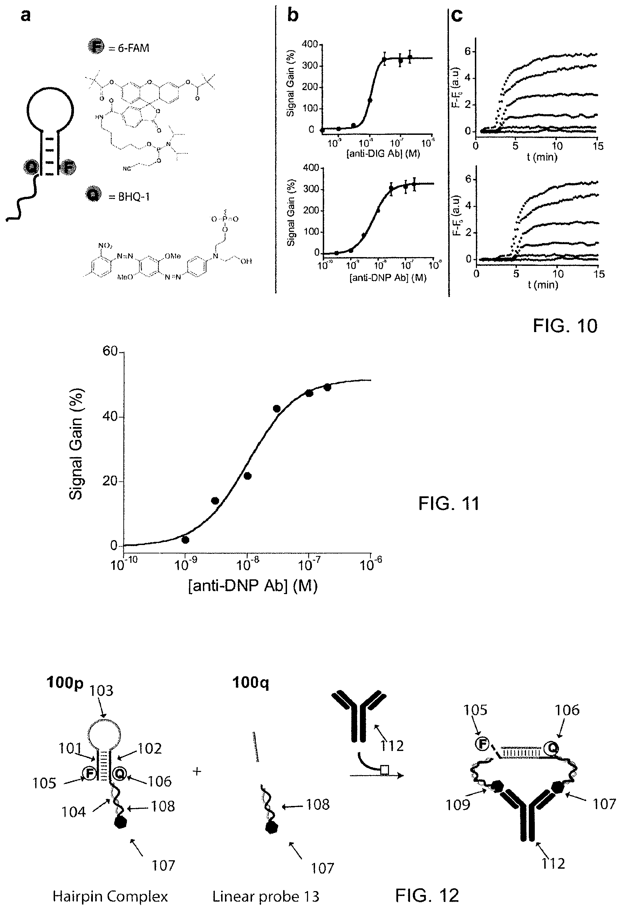

In other embodiments such as those described for instance using FIG. 12 there can be only hairpin probe (100p) coupled with the target binding moieties able to hybridize both with the signaling-coupled probe (or fluorophore/quencher, either electrochemical such as MB), and with the sequence 108 of the linear probe 13, in a molar ratio 2:1:1.

Advantageously, it is believed that systems and methods according to embodiments described herein proved to work in complex matrices such as blood and mucus, and moreover are modular and flexible because they can be made by various subunits that are connecting together in a target dependent manner. Moreover, the same sequences can be used to detect different targets and even multiple analytes in the same samples. Exploiting a target induced increase of the local concentration is possible to generate new structure not present before the addition of the target such as, DNA-RNA hybrid, DNA-PNA hybrid, that provide strategies that aim to increase the detection output signal through an amplification of the reaction. The steric hindrance of the binding moiety in this case does not limit the stability of a unique core structure compared to prior art WO-A-2012/071344. Moreover, the affinity and avidity of the target does not represent a limiting step of the nanoswitch activation because even a low affinity and avidity of the target is sufficient to generate an increase in the local concentration, the basic principle common to all these disclosed methods.

Below, the system and methods that include the oligonucleotide probes are described first in greater detail, followed by a review of the various methods in which the probes may find use, as well as a discussion of various representative applications in which the subject probes and methods my find use.

### SYSTEMS

Systems of the present disclosure include two or more oligonucleotide probes described in more detail below. The term "probe" as used herein refers to a biopolymer that undergoes a structural change upon its specific binding to a target (e.g., molecule, macromolecule, or analyte). Probes comprehend the following category of biological molecules: nucleic acids (DNA or RNA), non-natural oligonucleotide analogs such as peptide-nucleic-acid (PNA), locked-nucleic-acid (LNA), DNA or RNA based aptamers, peptides, polypeptides and proteins, etc. In some instances, the probes are oligonucleotides that may be of any length, but may be short oligonucleotides ranging from 15 to 100 nucleotides, or 25 to 90 nucleotides, such as 30 to 80 nucleotides. In some embodiments wherein the target binding moieties is an antibody or similar molecule, the length of the oligonucleotides may range from 20 to 600 nucleotides, in particular from 30 to 350. The particular use of terms "nucleic acid," "oligonucleotide," and "polynucleotide" should in no way be considered limiting and may be used interchangeably herein. "Oligonucleotide" is used when the relevant nucleic acid molecules include less than about 100 bases. "Polynucleotide" is used when the relevant nucleic acid molecules include more than about 100 bases. Both terms are used to denote DNA, RNA, modified or synthetic DNA or RNA (including but not limited to nucleic acids comprising synthetic and naturally-occurring base analogs, dideoxynucleotide or other sugars, thiols or other non-natural or natural polymer backbones), or other nucleobase containing polymers. Accordingly, the terms should not be construed to define or limit the length of the nucleic acids referred to and used herein.

Oligonucleotides of the present disclosure may be single-stranded, double-stranded, triple-stranded, or include a combination of these conformations. Generally, oligonucleotides contain phosphodiester bonds, although in some cases, as outlined below, nucleic acid analogs may have alternate backbones, comprising, for example, phosphoramide, phosphorothioate), phosphorodithioate, O-methylphosphoroamidite linkages, and peptide nucleic acid backbones and linkages. Other analog nucleic acids include those with positive backbones, non-ionic backbones, and non-ribose backbones. Nucleic acids containing one or more carbocyclic sugars are also included within the definition of nucleic acids. These modifications of the ribose-phosphate backbone may be done to facilitate the addition of additional moieties such as labels, or to increase the stability and half-life of such molecules in physiological environments. The term "nucleic acid sequence" or "oligonucleotide sequence" refers to a contiguous string of nucleotide bases and in particular contexts also refers to the particular placement of nucleotide bases in relation to each other as they appear in an oligonucleotide.

In certain embodiments, the probes may recognize their targets by specific binding of the target to the probe at, for example, a target binding moiety included on the probe. "Target" refers to any molecule that specifically binds to a probe of the present disclosure. These include, but are not limited to, macromolecules (e.g., proteins, carbohydrates, nucleic acids, lipids, inorganic macromolecules, nanostructure), small molecules (e.g., peptides, affybodies, aptamers, DNA-RNA-PNA probes, micro-RNA, piwi-RNA, circ-RNA, si-RNA, small-RNA). While not an exhaustive list, in certain embodiments, the target may be an antibody, a single-chain antibody, a DNA binding protein, a receptor, or an enzyme that specifically binds the probe. One of skill in the art will recognize that the important aspect of probe-target binding is not the particular mechanism involved but the fact that the binding is specific, as in specifically binding as defined in this disclosure.

In certain embodiments, the target is a bidentate target, where the complex may also be characterized by a polydentate bound. As used herein, "denticity" refers to the number of distinct binding sites included in a target molecule. A polydentate target may bind to two or more target binding moieties, with each target binding moiety binding to different binding sites on the target. For example, a bidentate target includes two target binding sites with each binding site capable of specifically binding to a target binding moiety. Bidentate targets may include, but are not limited to, antibodies which may include two antigen binding sites that each specifically bind to one copy of a specific antigen. In certain embodiments, the target is a non-bidentate target, for example a target that includes one binding site capable of specifically binding to a target binding moiety.

In some embodiments, described for instance using FIG. 5 and FIG. 6, systems and methods according to the present disclosure can be configured for performing a competitive strategy detection,. In possible implementations, the signaling moieties can be localized on different probes. In certain embodiments, the first signaling moiety is spatially far from the second signaling moiety, given that they are positioned on the two different probes. In this strategy, a competitor for the target is present of solution (e.g. an antibody for a specific antigen analyte). In the absence of target, the antibody brings to contact the two probes, and the distance of the first signaling moiety is held from the second signaling moiety is sufficient to minimize, suppress, or prevent the first signaling moiety from emitting a detectable signal; the signaling moieties become adjacent each other, by complementary base-pairing within the probe and increase in local concentration. In the presence of target, instead, the antibody is engaged with interaction with natural target that competes for its binding to the probes; probes remain at distance and this leads to a detectable signal that is directly proportional to the amount of the target.

For example, in embodiments, methods according to the present disclosure can be developed in a competitive format, in order to detect an antigen (see for instance FIG. 5). In such embodiments, the natural antigen to be detected competes with the antigen-labelled probes for the binding with the antibody. When the natural antigen is not present, the antibody interacts with the probes, and the quencher is displaced far from the fluorophore, switching on the signal. When the natural antigen is present, it prevents the antibody binding to the probes, therefore the stem loop remains in the closed configuration and the signal is switched off (FIG. 5).

In other competitive approach embodiments according to the present disclosure, the fluorophore is localized on the Hairpin Probe 3 (100j), whereas the quencher is localized on the Linear Probe 9 (1001) (FIG. 6). Therefore, when the natural antigene competes for the binding with the artificial antibody, the fluorophore on the hairpin probe is free to emit a signal, whereas when the natural antigene is not present in the sample, the antibody triggers the increase in local concentration between the probes and the formation of the hybridization between the hairpin complex and the linear probe 9; the fluorophore comes in proximity of the quencher, and is switched off (FIG. 6).

Embodiments of the system may also include a mix of different types of oligonucleotides such as nucleic acids (DNA or RNA), non-natural oligonucleotide analogs such as peptide-nucleic-acid (PNA), locked-nucleic-acid (LNA), DNA or RNA based aptamers.

In certain embodiments, a mixture RNA- and DNA-probes is exploited to increase the sensitivity of the system.

In one embodiment (FIG. 7) the Linear Probe 10 (100m) is biotinylated or coupled to magnetic nanoparticle, and is partially constituted by a RNA or PNA strand (122), which is complementary to the stem loop of the hairpin probe 100a. In such case target binding induces an increase of the local concentration of the probes and consequently the formation of hybrid duplex, DNA-RNA or DNA-PNA (126). An antibody, or another molecule recognizing specifically the hybrid duplex (127), conjugated with an enzyme (128), binds the duplex formation only when the target is present. The probe 100m can be immobilized in a streptavidin coated surface (if probe was biotinylated) or magnetic collection (if probe was couple to a magnetic nanoparticle) and a washing step to remove the excess of probe 100a. Adding specific substrate (129) the enzyme is able to start an enzymatic reaction measurable in terms of chemiluminescence, absorbance or through amperometry depending on the substrate and on the assay format (FIG. 7).

In certain embodiments described using FIG. 8, a nucleic acid intercalating dye (as for instance SYBR Green, SYTO9) can be added to the probe mixture with the aim to enhance the sensitivity of the system (FIG. 8). The dye, by its intrisic property, binds DNA only when it is double-stranded; and only when it binds DNA it is able to emit a fluorescent signal. This feature can be exploited to measure fluorescence coming from new double helix formation triggered by the increase in local concentration of the probes, or the decrease of the fluorescence signal caused from the melting of the double helix due to the dye release. Thus, the fluorescence signal coming from the binding of the target to the probes that move a fluorophore at a distance away from a quencher can be enriched adding signals coming from the incorporation or release of dyes in solution (FIG. 8).

Aspects of the present disclosure include oligonucleotide probes for detecting a target in a sample. The probes can be made as oligonucleotide strands constructed using techniques well-known to those of skill in the art, and include internal sequences allowing the oligonucleotide strand to undergo intramolecular hybridization when one internal hybridization sequence specifically hybridizes to a complementary internal hybridization sequence.

Intramolecular hybridization of the oligonucleotide probes can result in the probe taking a stem- loop secondary conformation in the absence of target binding to the probe. The probes are configured to use target binding-induced structural changes to detect the presence of the target in the sample. As used herein, the different oligonucleotide probe structures, such as those that exist in the presence or absence of a target, may be as referred to as "conformations." In certain embodiments, internal hybridization sequence lengths range from 5 to 25 nucleotides, for example 5 to 20 nucleotides, such as 10 to 20 nucleotides per internal hybridization sequence. The "loop" structures of each probe may be of any length suitable to the application, but may range from 3 to 30 nucleotides in length, for example 5 to 25 nucleotides, such as 10 to 20 nucleotides in length. In some embodiments multiple quenched fluorophores can be present on the same probe, where the probe is configured to use the target binding-induced structural changes to induce cooperatively conformational changes that activate all the fluorophore present on the probe.

The probes may be provided in solution. In these cases, the probes are free to diffuse through the solution and are not attached to a surface. In certain embodiments, the probes are attached to the surface of a substrate. The probes may be attached to the surface of the substrate at predetermined locations, such that the probes are arranged in an array formation. An "array," includes any one-dimensional, two-dimensional (as well as a three-dimensional) arrangement of addressable regions bearing a particular probe associated with that region. The probes may be covalently attached to the arrays at any point along the nucleic acid chain. In certain cases, the probes are attached at one of their termini (e.g., the 3' or 5' terminus). In some cases, the probes are attached to the array at an internal site of the probe. An "addressable array" includes any one or two dimensional arrangement of discrete regions (or "features") bearing particular probes associated with that region and positioned at particular predetermined locations on the substrate (each such location being at a known "address").

These regions may or may not be separated by intervening spaces.

Any given substrate may carry one, two, four or more arrays disposed on a front surface of the substrate. Depending upon the use, any or all of the arrays may be the same or different from one another and each may contain multiple spots or features. A typical array may contain more than ten, more than one hundred, more than one thousand, more than ten thousand features, or even more than one hundred thousand features, in an area of less than 20 cm², such as less than 10 cm². For example, features may have widths (that is, diameter, for a round spot) in the range from a 10 µm to 1.0 cm. In other embodiments each feature may have a width in the range of 1.0 µm to 1.0 mm, such as 5.0 µm to 500 µm, including 10 µm to 200 µm. Non-round features may have area ranges equivalent to that of circular features with the foregoing width (diameter) ranges. In certain embodiments, the arrays are formed by processes involving drop deposition of reagents, for example, photolithographic array fabrication processes may be used.

With arrays that are read by detecting fluorescence, the substrate may be of a material that emits low fluorescence upon illumination with the excitation light. Additionally, in this situation, the substrate may be relatively transparent to reduce the absorption of the incident illuminating light (e.g., laser light) and subsequent heating if the focused light travels too slowly over a region.

### Oligonucleotide Probes

In certain embodiments, the oligonucleotide probes are configured to produce a detectable signal when a target specifically binds to the probe to form a target-probe hybrid, or complex. The target may specifically recognize and bind to particular portions of the probe as, for example, a target binding moiety included on the probe. As used herein, the term "target binding moiety" refers to any molecule that specifically binds a target of the present disclosure. These include, but are not limited to, proteins, peptides, carbohydrates, nucleic acids, lipids, small molecules, and the like. For instance, the target binding moiety may be an antigen. The two or three probes include two target binding moieties, such as a first target binding moiety and a second target binding moiety. The first target binding moiety may be different from the second target binding moiety, for example in embodiments where the target is capable of binding to two or more different target binding moieties. In certain instances, the first target binding moiety and the second target binding moiety are substantially the same, for example in embodiments where the target is capable of binding two or more of the same target binding moiety, such as where the target is an antibody.

The target binding moiety may be bound to the probes. In some cases, the target binding moiety is directly bound to the probes. For example, the target binding moiety may be directly bound to the probe by modification of a nucleotide in the oligonucleotide strand that makes up the probe, such as, but not limited to, covalent attachment of the target binding moiety to a nucleotide in the oligonucleotide sequence, insertion of the target binding moiety between two nucleotides in the oligonucleotide sequence through the introduction of additional phosphodiester bonds, and the like.

In some cases, the target binding moiety is indirectly bound to the probes, such as, but not limited to, attachment of the target binding moiety to the probe through a linker moiety. The linker moiety can be any linker moiety suitable for the attachment of the target binding moiety to one or more nucleotides in the oligonucleotide probe. The linker moiety may include 1 to 25 carbons, such as 2 to 20 carbons, including 5 to 15 carbons. In certain embodiments, the target binding moiety is indirectly bound to the probe by hybridization of an oligonucleotide to the probe. In these embodiments, the target binding moiety may be attached either directly or indirectly to a hybridization sequence, which specifically hybridizes to a complementary sequence on the probe to form a duplex. As indicated above, certain embodiments of the probe include two target binding moieties. In some cases, the first target binding moiety and the second target binding moiety are attached to hybridization sequences that have different nucleotide sequences. In these cases, the hybridization sequences specifically hybridize to different complementary sequences on the probe. In other instances, the first target binding moiety and the second target binding moiety are attached to hybridization sequences that have substantially the same nucleotide sequence. In these instances, the hybridization sequences specifically hybridize to the same complementary nucleotide sequence. The probe may include one or more, such as two or more repeats of the complementary nucleotide sequence, such that a corresponding number of hybridization sequences may be hybridized to the probe, and thus a corresponding number of target binding moieties may be attached to the probe. In certain embodiments, the probe includes two hybridization sequences that are complementary to the hybridization sequences bound to the target binding moieties, such that two target binding moieties are attached to the probe.

In some embodiments, one of the probes also includes two or more hybridization sequences (e.g., intramolecular hybridization sequence, IHS) configured to allow the oligonucleotide strand to undergo intramolecular hybridization. For instance, the probe may include a first hybridization sequence (e.g., a first IHS) and a second hybridization sequence (e.g., a second IHS). The first hybridization sequence and the second hybridization sequence may be configured to form a duplex in the absence of target binding to both of the target binding moiety. The first hybridization sequence and the second hybridization sequence may be separated by a loop structure formed by the oligonucleotide sequence of the probe that is between the first hybridization sequence and the second hybridization sequence. As such, in the absence of target binding to the target binding moiety, the probe may adopt a stem-loop conformation.

In some embodiments, the probe may include two double-stranded regions separated by a single-stranded region. The single-stranded region may facilitate an increase in the flexibility of the probe in the absence of target binding.

The probes also include one or more signaling moieties. In some cases, the probe includes two signaling moieties, such as a first signaling moiety and a second signaling moiety. In certain embodiments, the first signaling moiety is held at distance in close proximity to the second signaling moiety, such as adjacent to the second signaling moiety, by complementary base-pairing within the probe. In some embodiments, the probe is flexible in the absence of target binding, allowing the signaling moieties to approach one another transiently or intermittently. In embodiments of the probe configured to produce a detectable change in signal in the presence of target binding to the target binding moieties, under conditions in the absence of target, the distance the first signaling moiety is held from the second signaling moiety is sufficient to minimize, suppress, or prevent the first signaling moiety from emitting a detectable signal. In some embodiments, this proximity instead enhances or maximizes the detectable signal from the first signaling moiety. In some embodiments, collisions between the two signaling moieties increase or decrease the signal or signals associated with them. When target is present and binds to the target binding moiety of the probe, the internal hybridization of the probe is disrupted because of the hybridization with the sequence of the other probe induced by the increase in local concentration. Disruption of the internal hybridization allows the end of the nucleotide chain to which the first signaling moiety is attached to move to a distance further away from the second signaling moiety. Under conditions in the presence of target, the distance the first signaling moiety moves away from the second signaling moiety is sufficient to lead to a detectable change in the signal from the first signaling moiety. In some embodiments, this change in distance leads to a detectable decrease in signal. In other embodiments, target binding prevents collisions between the two signaling moieties, leading to a detectable change in their signal.

As described above, in the absence of target binding to the target binding moiety, the signaling probe may be in a stem-loop configuration. In these cases, the probe may adopt a conformation where the first signaling moiety is positioned adjacent the second signaling moiety, such that the probe does not produce a detectable signal. For example, the first signaling moiety may be a fluorophore and the second signaling moiety may be a quencher. In these instances, under conditions in the absence of target, the distance the fluorophore is held from the quencher is sufficient to minimize, suppress, or prevent the fluorophore from emitting a detectable signal. Alternatively, this proximity may increase the signal from the first signaling moiety. When target is present and binds to the target binding moieties of the probes, the internal hybridization of the probe is disrupted because of the hybridization with the sequence of the other probe induced by the increase in local concentration. This causes the fluorophore to move to a distance further away from the quencher. Under conditions in the presence of target, the distance the fluorophore moves away from the quencher is sufficient to allow the signal emitted by the fluorophore to change detectably. In some instances, the detectable change in signal is an increase in the signal emitted by the fluorophore.

As described above, in the absence of target binding to the target binding moieties, the probe may have a flexible conformation. In these cases, the first signaling moiety can transiently collide with or bind to the second signaling moiety, such that the signal from the signaling moieties is changed. For example, the first signaling moiety may be a fluorophore and the second signaling moiety may be a quencher. In these instances, under conditions in the absence of target, collisions between the fluorophore and the quencher are sufficient to minimize, suppress, or prevent the fluorophore from emitting a detectable signal. When target is present and binds to both the target binding moieties of the probes, contact between the fluorophore and the quencher may be inhibited or reduced because of the hybridization with the sequence of the other probe induced by the increase in local concentration. This causes the quencher to not approach the fluorophore as readily or as frequently. Under conditions in the presence of target, the distance the fluorophore moves away from the quencher detectably changes the signal that the fluorophore emits. In certain cases, the detectable change in signal is an increase in the signal emitted by the fluorophore.

In some embodiments described in FIG. 9, the target is an antigen, and the target binding moieties present on Linear probe 11 and 12 (100o and 100p) may be constituted by molecules 138 and 140 that can be antibodies, or single-chain variable fragments (Sc-Fv), affybodies, aptabodies, Fab (Fragment antigen binding, and variants F(ab')2, F(ab') and chemically linked F(ab')2)), single domain antibodies. Such molecules 138 and 140 specifically bind the target in different epitopes. After hybridization between complementary sequences 139 of hairpin probe 4 (100n) and sequence 137 of probe 100o, an hairpin complex containing the molecules 138 is formed. Sequences 137, 139, 141 and 142, as well as sequences 101, 102 and 103 of the hairpin probe 4 (100n), can be constituted by 20 to 500 base pairs. Such molecules 138 and 140 can recognize different proximal epitopes of a specific antigen 143, and when antigen is present in the sample, it is recognized by the moieties 138 and 140; local concentration of the probes increases so that the hybridization between the Linear Probe 6 and Hairpin Complex generates the signal (FIG. 9).

### FLUOROPHORE

The term "fluorophore" refers to any molecular entity that is capable of absorbing energy of a first wavelength and re-emit energy at a different second wavelength. In certain embodiments, the oligonucleotide probe includes a fluorophore attached to one end of the probe or at a central position in the probe sequence, so long as the position of the fluorophore allows the fluorophore to be positioned adjacent the quencher in the absence of target binding to the target binding moieties and away from the quencher when target binds to the target binding moieties. In some embodiments, as discussed in more detail below, the fluorophore may be attached to one end of the probe. The fluorophore attached to the probe need not be a single molecule, but may include multiple molecules. In some embodiments, the fluorophore is a fluorescent moiety, such as but not limited to, a fluorescent nanoparticle, such as gold, silver or diamond nanoparticles, quantum dot, and similars. The "end" of the oligonucleotide probe possessing the fluorophore includes any nucleotide within one quarter of the total number of nucleotides in the probe from the terminal nucleotide. Alternatively, the end possessing the fluorophore includes the terminal 10, 9, 8, 7, 6, 5, 4, 3 or 2 nucleotides of the probe. Attachment may also be on the terminal nucleotide alone. The attachment of the fluorophore to the oligonucleotide probe allows the fluorophore to be positioned in an alternate configuration at a distance away from the quencher in response to target specifically binding the probe, thereby generating a detectable signal.

The fluorophore may be synthetic or biological in nature, as known to those of skill in the art. More generally, any fluorophore can be used that is stable under assay conditions and that can be sufficiently suppressed when in close proximity to the quencher such that a significant change in the intensity of fluorescence of the fluorophore is detectable in response to target specifically binding the probe. Examples of suitable fluorophores include, but are not limited to CAL Fluor Red 610 (FR610; Biosearch Technologies, Novato, CA), fluorescein isothiocyanate, fluorescein, 6-carboxyfluorescein (6-FAM), rhodamine and rhodamine derivatives, coumarin and coumarin derivatives, cyanine and cyanine derivatives, Alexa Fluors (Molecular Probes, Eugene, OR), DyLight Fluors (Thermo Fisher Scientific, Waltham, MA), and the like.

The term "quencher" may refer to a substance that absorbs excitation energy from a fluorophore and dissipates that energy as heat. The quencher may also absorb excitation energy from a fluorophore and dissipate that energy as re-emitted light at a different wavelength.

Quenchers are used in conjunction with fluorophores, such that when the quencher is positioned adjacent the fluorophore or at a distance sufficiently close to the fluorophore, the emission of the fluorophore is suppressed. However, when the quencher is positioned away from the fluorophore or at a distance sufficiently far from the fluorophore, the emission of the fluorophore is not suppressed, such that a signal of the fluorophore is detectable. Alternatively, the quencher may include moieties that reduce the emission of the fluorophore via photoelectron transfer, resonance energy transfer or other quenching mechanisms. The quencher may also be replaced by a second fluorophore capable of resonance energy transfer, by a second fluorophore capable of forming an excimer or exiplex or, in general, by any other group that modulates the fluorescence of the first fluorophore.

The oligonucleotide probes may include a quencher attached at a central position away from the ends of the probe (e.g., at a position in the central portion of the probe sequence) or at one end of the probe, as long as the position of the fluorophore allows the fluorophore to be positioned adjacent to the quencher in the absence of target binding to the target binding moieties and away from the quencher when target binds to the target binding moieties. The quencher attached to the probe need not be a single molecule, but may include multiple molecules. The attachment position of the quencher includes any nucleotide within the probe that positions the quencher in close proximity to the fluorophore in the absence of target specifically binding to the target binding moieties. The attachment of the quencher to the oligonucleotide probe allows the quencher to be positioned in an alternate configuration at a distance away from the fluorophore in response to target specifically binding the probe, thereby detectably changing the signal emitted by the fluorophore. In certain instances, the detectable change in the signal is an increase in the signal emitted by the fluorophore.

The quencher may be synthetic or biological in nature, as known to those of skill in the art. More generally, any quencher can be used that is stable under assay conditions and that can sufficiently suppress the fluorescence of the fluorophore when in close proximity to the fluorophore such that a significant change in the intensity of fluorescence of the fluorophore is detectable in response to target specifically binding the probe. Examples of quenchers include, but are not limited to, Black Hole Quencher (BHQ; Biosearch Technologies, Novato, CA), Dabsyl (dimethylaminoazosulphonic acid), Qxl quenchers (AnaSpec Inc., San Jose, CA), Iowa black FQ, Iowa black RQ, and the like. In another embodiment the quencher may also be fluorescent, leading to emission at a second wavelength when the quencher is in proximity to the first fluorophore. Examples of such fluorophore/quencher pairs include Alexa488-Alexa555, Alexa488-Cy3, Cy3-Cy5. In other embodiments, the quencher is a second fluorophore that forms an excimer or an exciplex with the first fluorophore, leading to a change in fluorescence upon their segregation. An example would include an embodiment in which both the fluorophore and the quencher are pyrene.

In certain embodiments, the probes of the present disclosure are oligonucleotides that include a first signaling moiety that includes a macromolecule having a catalytic activity and a second signaling moiety that includes an inhibitor (or an activator) of the catalytic activity. In certain embodiments, the catalytic macromolecule is held at distance in close proximity to the inhibitor, such as adjacent the inhibitor, by complementary base-pairing within the probe. In embodiments of the probe configured to produce a detectable change in signal in the presence of target binding to the target binding moieties, under conditions in the absence of target, the distance the catalytic macromolecule is held from the inhibitor is sufficient to minimize, suppress, or prevent the catalytic macromolecule from performing its catalytic activity. In some embodiments, such as where the second signaling moiety is an activator, this proximity instead enhances or maximizes the catalytic activity of the catalytic macromolecule. When target is present and binds to the target binding moieties of the probes, the internal hybridization of the probe is disrupted because of the hybridization with the sequence of the other probe induced by the increase in local concentration. Disruption of the internal hybridization allows the end of the nucleotide chain to which the catalytic macromolecule is attached to move to a distance further away from the inhibitor. Under conditions in the presence of target binding, the distance the catalytic macromolecule moves away from the inhibitor is sufficient to lead to a detectable change in the catalytic activity of the catalytic macromolecule. In some embodiments, this change in distance leads to a detectable increase in signal.

According to possible implementations, the macromolecule having a catalytic activity may be HRP (Horseradish Peroxidase enzyme), alkaline phosphatase or GOD (Glucose Oxidase enzyme) with the respective inhibitor, which can be a protein, peptide, aptamer or chemical inhibitor.

In certain embodiments, the target may be removed and the probe regenerated using mild conditions that retain the integrity of the probe and allow the probe to re-establish the internal base pair hybridization pattern that suppresses the fluorescence of the fluorophore. In these embodiments, the probes are reusable, such that the probes may be regenerated as described above and reused any number of times, such as 2 or more times, including 3 or more times, for instance 5 or more times, or 10 times or more, while maintaining substantially the same ability to detect a target in a sample.

In certain embodiments, the probes are capable of specifically identifying nanomolar or picomolar concentrations of targets in a sample. For example, the probes may be configured to detect a target in a sample, where the target has a concentration ranging from 1 pM to 100 nM, such as from 1 pM to 750 pM, including from 5 pM to 500 pM, or from 10 pM to 300 pM. In some instance the probes may be configured to detect a target in a sample, where the target has a concentration ranging from 1 nM to 1 µM, such as from 1 nM to 750 nM, including from 1 nM to 500 nM, or from 1 nM to 250 nM, for instance from 1 nM to 100 nM.

The phrase binding "specifically" or "selectively," refers to the interaction of an oligonucleotide probe, as described herein, with a specific target in a manner that is determinative of the presence of the target in the presence or absence of a heterogeneous population of molecules that may include nucleic acids, proteins, and other biological molecules. Thus, under designated conditions, a specified oligonucleotide probe binds to a particular target and does not bind in a significant manner to other molecules in the sample. Probes do not bind to a molecule in a detectable or significant manner when the interaction does not disrupt the intramolecular hybridization of the probe resulting in no significantly detectable signal or no significantly detectable change in signal from the probe.

Moreover, "specific binding" results in a disruption of intramolecular hybridization between probe nucleotide sequences resulting in a conformational change in the probe such that the probe produces a detectable signal or a detectable change in a signal. Thus, specific binding may be determined by titration of the probe with a target. Specific binding will allow an increase (or decrease) in signal with increasing amount of target contacted with the probe.

### Probes that use binding-induced segregation of two target binding moieties

An example of the oligonucleotide probes 100g (hairpin probe 2), 100h (linear probe 6) and 100i (linear probe 7) configured to produce a detectable signal upon target binding-induced segregation of two target binding moieties is depicted in FIG. 3.

An aspect of the oligonucleotide probe of FIG. 3 is that the hairpin probe 100g has a stem-loop structure formed by intramolecular hybridization of a single-stranded oligonucleotide. The stem-loop structure of the probe 100g is formed through intramolecular hybridization between a first hybridization sequence 101 and a second hybridization sequence 102. Internal hybridization between first hybridization sequence 101 and second hybridization sequence 102 forms a duplex. First hybridization sequence 101 and second hybridization sequence 102 are separated by a loop sequence 103 formed by the oligonucleotide sequence between first hybridization sequence 101 and second hybridization sequence 102. The oligonucleotide probe 100g also includes a first target binding moiety 104.

The oligonucleotide probe further includes a fluorophore 105 and a quencher 106. In FIG. 1 and FIG. 3 the fluorophore 105 is coupled to one end of the oligonucleotide strand of the probe and the quencher 106 is coupled internally of the oligonucleotide strand of the probe.

The linear probe 6 (100h) includes a first target binding moiety 107 and a hybridization sequence 108 which is complementary to the hybridization sequence 104 of probe 100g; the complex between the hairpin probe (100g) and the linear probe 6 (100h) is defined as hairpin complex. The first target binding moiety 107 may also directly be attached to the hairpin probe 100a at the end of the tail sequence 104 as described above (FIG. 1).

The linear probe 7 (100i) includes a second target binding moiety 109 and a first hybridization sequence 110 that acts as a linker between the second target binding moiety 109 and a second hybridization sequence 111. The probe 100i also includes a second hybridization sequence 111 which is complementary to the loop sequence 103 of the hairpin probe 100g.

As shown in FIG. 3, in the absence of target binding to the target binding moieties (107 and 109), the internal hybridization between the first and second hybridization sequences (101 and 102) positions the fluorophore 105 adjacent the quencher 106, such that the quencher 106 substantially suppresses detectable emissions from the fluorophore 105 (see FIG. 10).

Also, in the absence of target binding to the target binding moieties (107 and 109), the hybridization between the hybridization sequence 108 of the probe 100h and the hybridization sequence 104 of the probe 100g forms a stable scaffold system.

Also, in the absence of target binding to the target binding moieties (107 and 109), the hybridization between the hybridization sequence 111 of the probe 100h and the loop sequence 103 of the probe 100g is not possible due to the competing effect of the stem-loop structure that prevents hybridization.

As shown in FIG. 3, binding of the target 112 (e.g., an antibody) to the first and second target binding moieties (107 and 109) induces a dramatic increase in the local concentration of the hybridization sequence 111 of the probe 100i and the loop sequence 103 of the hairpin complex made by hairpin probe 2 100g and linear probe 2 100h. This increase in local concentration induces the hybridization between the two sequences (103 and 111) and causes a conformational change in the hairpin probe 100g that positions the fluorophore 105 at a distance away from the quencher 106, such that the fluorophore 105 produces a detectable signal (see FIG. 10).

In certain examples, the length of each stem duplex structure may be different, as is also the case with loop structures in the probe 100a. Similarly, the length of each hybridization sequence of probes 100h and 100i may be different. Limits on the size of each duplex, each loop, and the single-stranded linear probe length are not contemplated as being rigidly limited but are rather application dependent. Optimal lengths for each of the probe components described herein may be determined without undue experimentation by one of skill in the art through the teachings of this specification. Lengths provided herein are examples only.

### Probes Configured to Produce Other Types of Signals upon Target Binding

In certain embodiments, the probe is configured to produce a signal change through different signal output mechanisms. In some cases, the probe includes two target binding moieties, such as a first target binding moiety and a second target binding moiety. Various signaling moieties may be used, where the first and second signaling moieties produce a detectable signal change upon target binding. The detectable change in signal includes, but is not limited to, a detectable signal decrease when a target specifically binds to the probe to form a target-probe hybrid, or complex, or a detectable increase in signal when a target specifically binds to the probe to form a target-probe hybrid, or complex. In certain embodiments, the first signaling moiety is a detectable reporter and the second signaling moiety is a detector configured to detect the reporter. Suitable reporters may include reporters that are detectable by the detector, such as, but not limited to, electrochemical reporters, magnetic reporters, and the like.

According to the invention (FIG. 2), the first signaling moiety is an electrochemical reporter and the second signaling moiety is an electrode. In some cases, the use of an electrochemical reporter and an electrode as the signaling moieties may facilitate target detection directly in whole blood or other complex clinical, food and environmental samples.

As represented in FIG. 2, the linear probe 100c contains an electrochemical reporter 113 and also contains a hybridization sequence (114).

Another linear probe 100d contains the first target binding moiety 115 and two hybridization sequences 116 and 117. The second hybridization sequence 117 is complementary to the hybridization sequence (114) of the probe 100c.

The first target binding moiety 115 of probe 100d may be directly or indirectly attached to the probe as described above. The hybridization between the hybridization sequence 114 of the probe 100c and the hybridization sequence 117 of the probe 100d is such that the formation of the complex between probe 100c and 100d is very stable.

Another probe 100e includes a second target binding moiety 118 and a hybridization sequence 119. The hybridization sequence 119 is such that can hybridize to the two hybridization sequences 116 and 117 of probe 100d.

Finally, a probe 100f contains a modified nucleotides configured to be attached to the surface of the substrate, such as, but not limited to nucleotides modified to include a thiol group. The probe may be attached to the surface of the substrate directly, such as by covalent attachment of the probe to the surface of the substrate, or indirectly, such as through a linker moiety or by affinity binding (e.g., through streptavidin-avidin complex formation, and the like). The probe 100f also includes a hybridization sequence 120. The hybridization sequence 120 is such that can hybridize to the hybridization sequence 114 of probe 100c. The probe 100f is immobilized onto the surface of an electrode.

As shown in FIG. 2, in the absence of target binding to the target binding moieties (115 and 118), the hybridization between the second hybridization sequences (117) of the probe 100d and the probe 100e make a stable complex. Because of the relatively low concentration of the probes the system is stable and the reporter conjugated probe (100c) cannot be released from the probe 100d.

As shown in FIG. 2, binding of the target 121 (e.g., an antibody) to the first and second target binding moieties 115 and 118 induces a dramatic increase in the local concentration of the hybridization sequence 116 of the probe 100d and the hybridization sequence 119 of the probe 100e. This increase in local concentration induces the hybridization rate between the two sequences 116 and 119 and, through a strand displacement mechanism causes the release of the reporter conjugated probe 100c from the complex and the perfect matched binding between probe 100d and probe 100e. The reporter conjugated probe 100c can thus hybridize to the probe 100f and produce a detectable electrochemical signal (see FIG. 2, lower panels).

In certain examples, the length of each duplex structure may be different. Similarly, the length of each hybridization sequence of probes 100c, 100d, 100e and 100f may be different. Limits on the size of each duplex and the single-stranded linear probe length are not contemplated as being rigidly limited but are rather application dependent. Optimal lengths for each of the probe components described herein may be determined without undue experimentation by one of skill in the art through the teachings of this specification. Lengths provided herein are examples only.

In other embodiments, the electrochemical reporter is directly conjugated to probe 100f so that in the absence of the target a high signal is produced. The release of the strand 100c induced by the target binding of the probe 100d causes the hybridization of the strand 100c with the strand 100f. This causes that the electrochemical reporter is held a distance away from the electrode, such that a detectable signal is not produced or a low detectable signal is produced.

In some instances, the probe may be attached to the surface of a substrate. As described above, the first signaling moiety may be an electrochemical reporter and the second signaling moiety may be an electrode. As such, the probe may be attached to the surface of the electrode. The probe may be attached by any convenient attachment method suitable for attachment of the oligonucleotide probe to the surface of the substrate. For example, the probe may include modified nucleotides configured to be attached to the surface of the substrate, such as, but not limited to nucleotides modified to include a thiol group. The probe may be attached to the surface of the substrate directly, such as by covalent attachment of the probe to the surface of the substrate, or indirectly, such as through a linker moiety or by affinity binding (e.g., through streptavidin-avidin complex formation, and the like).

It shall be noted that, advantageously, in all embodiments described herein, the approach is much simpler than the strategy of WO'029, given that there is a single recognition time (the analyte that recognizes the binding moieties) that is enough to trigger the signal. The features of the proposed method according to embodiments described herein have strong implications in the rapidity and the robustness of the sensing system, given that the signal will be based only on the affinity of a single probe pair, and not on the combination of affinities of multiple elements such as it occurs in WO'029.

### METHODS

### Detection of Targets Using Oligonucleotide Probe-Based Detectors

Provided are methods for detecting the presence of a target in a sample using oligonucleotide probe-based detectors. Aspects of the methods include contacting a sample suspected of containing a target with two or more probes of the present disclosure under conditions that allow target that may be present in the sample to specifically bind to the target binding moieties of the probes. Binding of the target to the probes causes an increase of local concentrations of the probes that induces a reaction between the probes that causes a conformational change in the signaling probe, which in turn produces a detectable signal or a detectable change in a signal. For example, binding of the target to the probes may position a fluorophore at a distance away from a quencher sufficient to allow a signal of the fluorophore to be detectable. In other embodiments, binding of the target to the probe may position a reporter moiety close to a detector sufficient to allow a change in a signal from the detector to be detectable.

The detectable signal or the detectable change in signal may be compared to control readouts from control samples that do not contain target or to results from samples that contain targets that do not specifically bind to the target binding moieties of the probe (e.g., negative controls). In other embodiments, the signal detected by the detector may be optionally compared to control readouts for control samples that contain target or a known amount of target (e.g., positive controls). Numerous alternative controls may be performed individually or in combination, as is known to those of skill in the art. For example, the control may be to challenge the probe with a surrogate solution absent the sample, and thus lacking target.

Alternatively, the control may be a solution containing a target derivative that may have similarity to the actual target, but is normally not recognized and specifically bound by the probe under specific binding or "stringent" conditions.

Suitable samples include bodily fluids (e.g., blood, urine, interstitial fluid, lachrymal fluid, sweat, saliva, and the like), water, cell extracts, cell suspensions, secretions, solvents, and other aqueous and organic liquid solutions, suspension or emulsions capable of including the target of the probe of the detector. Samples may also include complex samples, such as, but not limited to, whole blood, crude nuclear extracts, and the like. In certain embodiments, the probes of the present disclosure are oligonucleotides that include target binding moieties, such as antigens, that specifically bind to target antibodies. In certain embodiments, the probes of the present disclosure are oligonucleotides that include target binding moieties, such as polypeptides, that specifically bind to target macromolecules. In certain embodiments, the probes of the present disclosure are oligonucleotides that include target binding moieties, such as aptamers, that specifically bind to target macromolecules.

### Reaction Conditions and Detection Methods

The methods disclosed herein may be carried out in any reaction medium that allows specific binding between probe and, if present, target as defined herein.

Binding reactions involving the probes disclosed herein may be carried out in the presence of agents and additives that promote the desired specific binding, diminish nonspecific background interactions, inhibit the growth of microorganisms, or increase the stability of the probe and/or target. Binding reactions of the disclosure may be carried out at ambient temperature, although any temperature in the range allowing specific binding may be used. For instance, in some embodiments, the temperature range is from 5 °C to 45 °C, such as from 10 °C to 40 °C, or from 20 °C to 30 °C. In addition, in some embodiments, the pH of the binding reaction medium is about physiological pH. For example, the pH may range from 4 to 10, such as 5 to 9, including 6 to 8. In certain cases, the pH may be 7. For convenience, reaction conditions may be chosen to allow specific binding to occur as rapidly as possible. Binding times as short as seconds (e.g., 1 to 60 seconds), or minutes (e.g. 1 to 30 minutes) may be employed. By way of example, times of 1 to 60 seconds, such as 10 to 60 seconds, including 20 to 60 seconds may be used. In other embodiments, times of 1 to 30 minutes, such as 5 to 20 minutes, including 10 to 20 minutes may be used.

### Multiplexing

In certain embodiments, the methods may include multiplex detection of targets. The terms "multiplex" or "multiplexing" as used herein refer to using multiple distinct signaling moieties, such that a single assay may be used to detect the presence of different targets in a single sample.

For example, in embodiments that include fluorescent signaling moieties, the system may include multiple fluorescently distinct fluorophores, such that a single assay may include multiple probes each with different fluorophores. Fluorophores of these embodiments emit detectable signals at different wavelengths. Multiplexing facilitates the detection of different targets of interest within a single sample (e.g., targets that specifically bind to different target binding moieties). In these embodiments, a mixture of differentially labeled probes (e.g., a first probe with a first fluorophore and a second probe with a second fluorophore) may be contacted with a sample that includes one or more different targets of interest. Both the signal of the first and second fluorophores may be detected independently, thus indicating the presence (or absence) of the first target and the second target in the sample. In certain embodiments, multiplexing may be used in systems comprising arrays or addressable arrays of probes attached to a substrate surface.

Another multiplex approach may include multiple probes with different target binding moieties, labelled with same fluorescence. The different moieties are specific for one target (e.g. several epitopes of the same antigen, linear and/or conformational) and the signal that will be generated will be the sum of all the signal from each target. This multiplex system allows to amplify the depth of the analysis.

Similarly, multiplexing may be applied to embodiments that include electrochemical signaling moieties. In these embodiments, two or more different probes may be contacted with a sample that includes one or more different targets of interest. For example, the system may include a first probe that includes a first and a second target binding moieties and a second probe with a third and a fourth target binding moieties. The first and the second target binding moieties may be different from the third and the fourth target binding moieties, such that a first target binds to the first and second target binding moieties and a second target binds to the third and fourth target binding moieties. Upon binding of the first target to the first and second target binding moieties of the first probe, a conformational change is induced in the first probe such that the first probe produces a detectable change in a first signal. In addition, upon binding of the second target to the third and fourth target binding moieties of the second probe, a conformational change is induced in the second probe such that the second probe produces a detectable change in a second signal that is distinct from the first signal. The changes in the first and second signals may be detected, thus indicating the presence (or absence) of the first target and the second target in the sample.

Thanks to their modularity and simplicity, the embodiments according to the present disclosure are more suitable for multiplexing analysis.

### UTILITY

The subject systems and methods find use in a variety of different applications where determination of the presence or absence, and/or quantification of one or more targets in a sample is desired. In certain embodiments, the methods are directed to the detection of proteins, carbohydrates, nucleic acids, lipids, peptides, enzymes or other biomolecules in a sample.

Samples may include, but are not limited to, blood, plasma, serum, or other bodily fluids or excretions, such as but not limited to, urine, saliva, semen, prostatic fluid, nipple aspirate fluid, lachrymal fluid, perspiration, feces, cheek swabs, cerebrospinal fluid, cell lysate samples, amniotic fluid, gastrointestinal fluid, biopsy tissue, and the like.

The presence or absence of a target in a sample or significant changes in the concentration of a target over time can be used to diagnose disease risk, presence of disease in an individual, or to tailor treatments for the disease in an individual. For example, the presence of a particular target or panel of targets may influence the choices of drug treatment or administration regimes given to an individual. In evaluating potential drug therapies, the presence, absence, or concentration of a target may be used as a surrogate for a natural endpoint such as survival or irreversible morbidity. If a treatment alters the target, which has a direct connection to improved health, the target can serve as a surrogate endpoint for evaluating the clinical benefit of a particular treatment or administration regime. Thus, personalized diagnosis and treatment based on the particular target or panel of targets detected in an individual are facilitated by the subject systems and methods. Furthermore, the early detection of targets associated with diseases is facilitated by the high sensitivity of the subject systems and methods, as described above. Due to the multiplex capability of detecting multiple targets in a single assay, combined with selectivity, sensitivity and ease of use, the presently disclosed systems and methods find use in quantitative, point-of-care or near-patient bio-molecular assays.

The subject systems and methods find use in diagnostic assays, such as, but not limited to, the following: detecting and/or quantifying targets, as described above; screening assays, where samples are tested at regular intervals for asymptomatic subjects; prognostic assays, where the presence and or quantity of a target is used to predict a likely disease course; stratification assays, where a subject's response to different drug treatments can be predicted; efficacy assays, where the efficacy of a drug treatment is monitored; efficacy of a vaccine and its monitoring; and the like.

The subject devices, systems and methods also find use in validation assays. For example, validation assays may be used to validate or confirm that a potential disease biomarker is a reliable indicator of the presence or absence of a disease across a variety of individuals. The short assay times for the subject systems and methods may facilitate an increase in the throughput for screening a plurality of samples in a minimum amount of time.

In certain embodiments, the subject systems and methods find use in detecting antibodies in a sample. In some cases, the subject systems and methods may be used to detect the presence or absence of particular antibodies, as well as an increase or decrease in the concentration of particular antibodies in a sample.

### KITS

Also provided are kits that find use in practicing the subject methods, as described above. For example, kits and systems for practicing the subject methods may include one or more systems of the present disclosure, which may include one or more probes. As such, in certain embodiments the kits may include a solution or suspension of the probes in an aqueous or other compatible solution. The one or more probes may be provided in separate containers with each container including a single type of probe, or may be provided in a container that includes a mixture of two or more types of probes. In other embodiments, the kits may include one or more probes immobilized on the surface of a substrate forming an addressable array of probes as described above.

In addition to the above components, the subject kits may further include instructions for practicing the subject methods.

As can be appreciated from the disclosure provided above, the present disclosure has a wide variety of applications. Accordingly, the following examples are offered for illustration purposes and are not intended to be construed as a limitation on the invention in any way. Those of skill in the art will readily recognize a variety of noncritical parameters that could be changed or modified to yield essentially similar results. Thus, the following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric.

### EXAMPLES

### Fluorescent Probes

### Fluorescent probes for antibody detection

The first set of probes was tested by using a pair of probes containing as target binding moiety the hapten 2,4-dinitrophenol (DNP) and thus targeting anti-DNP antibodies were used (FIG. 2). The DNP target binding moieties were covalently attached to the probes via the introduction of additional phosphodiester bonds (see Material and Methods below). As signaling probe a sequence that includes a stem-loop with a relatively strong stem-loop conformation was used. A 6-carboxyfluorescein (6 FAM) and Black Hole Quencher 1 (BHQ-1) were attached to the 5' end and internally in the signaling probe to produce a detectable fluorescent signal upon opening of the stem-loop structure of the signaling probe.

The second set of probes included a pair of probes containing as target binding moiety the hapten digoxigenin (Dig) and thus targeting anti-Dig antibodies were used (FIG. 10). The Dig target binding moieties were covalently attached via amine linkers on the C-5 positions of the two thymines in the middle of the double- stranded stem (see Material and Methods below). As signaling probe a sequence that includes a stem-loop with a relatively strong stem-loop conformation was used. A 6-carboxyfluorescein (6 FAM) and Black Hole Quencher 1 (BHQ-1) were attached to the 5' end and internally in the signaling probe to produce a detectable fluorescent signal upon opening of the stem-loop structure of the signaling probe.

The anti-Dig and anti-DNP antibody probes produced 330% and 312% increases in fluorescence, respectively, at saturating target concentrations (FIG. 10(b)). Both probes responded to their respective target antibodies at 10 nM (FIG. 10(b)), and no increase in fluorescence was observed when the two antibodies were interchanged (FIG. 10(b), dotted line). The two probes displayed low-nanomolar affinities and equilibration times of one minute or less (FIG. 10(c)), and achieved detection limits of 3 nM and 4 nM (e.g., 450 and 675 ng/ml) in less than one minute for anti-Dig and anti-DNP antibody probes, respectively (FIG. 10(b)). The titrations and kinetic traces shown in FIG. 10 represent the average of at least three independent measurements, with error bars reflecting the average absolute deviation.

Probes were also tested in a competitive assay format in order to detect small molecules such as DNP. The DNP-signaling probes (at a concentration of 20 nM) were used to detect the presence of free DNP (FIG. 5). An anti-DNP antibody was first incubated with free DNP in the sample and then after a specific incubation time (10 minutes) the set of probes for Anti-DNP antibody detection was added to detect the unbound anti-DNP antibody and thus, in a indirect way, the free DNP in the sample.

Experiments were also performed to test fluorescent probes for the detection of targets directly in blood serum. FIG. 11 shows the detection of DNP- antibodies directly in blood serum at 37 °C using a set of probes containing as signaling moieties Alexa Fluor-680 for the fluorophore and BHQ-2 for the quencher (blood serum has a low fluorescence in the infrared).

### Electrochemical Probes

### Electrochemical probes for antibody detection

The approach can also be used for the electrochemical detection of the same bioanalytical targets. In this case the signaling probe contains a redox reporter (e.g. methylene blue). A set of two probes modified with target recognition tags were also used. The binding of the antibody to these two recognition elements increases the local concentration of the two probes and thus triggers the release of the redox-reporter-modified probe. This redox-reporter-modified probe is recognized by a thiol-modified probe on the surface of an electrode thus positioning the methylene blue electrochemical reporter in proximity to the electrode, promoting electron transfer and producing an increase in the faradaic current.

Electrochemical probes detected nanomolar concentrations of their targets.

A detectable increase in the current signal from anti-Dig antibodies was observed.

The anti-Dig probes achieved 3 nM detection limits (e.g., 450 to 650 µg/ml), respectively (FIG. 2), which was well below the serum concentrations typical of antibodies. The electrochemical probes did not measurably respond to non-targeted monoclonal or polyclonal antibodies at 30 nM even when mixed with a 3 µM mixture of random human antibodies. The titrations traces shown in FIG. 2 represent the average of measurements conducted with at least four independently fabricated sensors, with error bars reflecting the average absolute deviation. Shown in FIG. 2 lower panels are square wave voltammograms for anti-Dig antibody.

### Material and methods

Fluorescent and DIG/DNP modified probes: HPLC purified DNAs modified with 5' - FAM, 3'-BHQ-1 (for in serum measurement 5'-Alexafluo680 and 3'-BHQ2) and either Dig or DNP were purchased from IBA (Goettingen, Germany) and Biosearch Technologies (Novato, CA), respectively. All fluorescent constructs possessed an additional thymine base after the FAM-and thymine nucleotide before the BHQ-1. Dig and DNP were inserted on a thymine modified nucleotide at the 5'- or 3'- end of the sequence:
Fluorescent probe (anti-DIG antibodies):
   Hairpin probe 2: 5'- (FAM)TAGGCGTTCTAGATATGCAATCGCCTT (BHQ1)GTCTCAGAATGTAAG-3'
   wherein TAGGCGTTCTAGATATGCAATCGCCTT is SEQ ID NO. 1 and GTCTCAGAATGTAAG is SEQ ID NO. 2.
DIG-modified probes:
   Linear probe 6: 5'- (DIG) TTTTT CTTACATTCTGAGAC -3' wherein TTTTT CTTACATTCTGAGAC is SEQ ID NO. 3.
   Linear probe 7: 5'-(DIG) TTTTTTTTTTTTTTT ATTGCATATCTAGAA-3' wherein TTTTTTTTTTTTTTT ATTGCATATCTAGAA is SEQ ID NO. 4.
Fluorescent probe (anti-DNP antibodies):
   Hairpin probe 2: 5'- (FAM or ALEXA680)TGAGCGGTATAGCCTAATTCGCGCTCT(BHQ1 or BHQ2)CTGTCACTTTCTGAG -3'
   wherein TGAGCGGTATAGCCTAATTCGCGCTCT is SEQ ID NO. 5 and CTGTCACTTTCTGAG is SEQ ID NO. 6.
DNP-modified probes:
   Linear probe 6: (DNP) TTTTT CTCAGAAAGTGACAG-3'
   wherein TTTTT CTCAGAAAGTGACAG is SEQ ID NO. 7.
   Linear probe 7: (DNP)TTTTTTTTTTTTTTT CGAATTAGGCTATAC-3'
   wherein TTTTTTTTTTTTTTT CGAATTAGGCTATAC is SEQ ID NO. 8.
Electrochemical probes for antibody detection: HPLC purified DNA and modified with internal C6-thiol and 3'-terminal methylene blue (MB) was purchased from Biosearch Technologies (Novato, CA);
Linear Probe 3 probe: 5'-TCT CAA GAT CCT AGG TAA GTC GTT TTT TTT TT (Dig)-3'
   wherein TCT CAA GAT CCT AGG TAA GTC GTT TTT TTT TT is SEQ ID NO. 9. Linear Probe 2: 5'- CTT ACC TAG GAT CTT GAG A (Methylen Blue) -3'
   wherein CTT ACC TAG GAT CTT GAG A is SEQ ID NO. 10.
   Linear Probe 4: CGA CTT ACC TAG GAT CTA ATT TTT TTT TTT TT (Dig)-3';
   wherein CGA CTT ACC TAG GAT CTA ATT TTT TTT TTT TT is SEQ ID NO. 11. Linear probe 5 5'-(HS-C6)-TCTCAAGATCCTAGG-3'
   wherein TCTCAAGATCCTAGG is SEQ ID NO. 12.

Antibodies were purchased from Roche Diagnostic Corporation (Mannheim, Germany) (Sheep polyclonal anti-digoxigenin and its Fab fragments), Sigma-Aldrich (St. Louis, MO) (Mouse Monoclonal Anti-DNP). Heparinized whole blood (bovine calf) was purchase from Innovative Research (Michigan, USA).

All fluorescent experiments were conducted at pH 7 in 50 mM sodium phosphate buffer, 150 mM NaCl at 25 °C, unless otherwise indicated. Equilibrium fluorescence measurements were obtained using a Cary Eclipse Fluorimeter with excitation at 480 (± 5) nm and acquisition at 517 (± 5) nm. Fluorescence spectra were obtained using 10 nM solutions of each probe. Binding curves were obtained using 10 nM of probes by sequentially increasing the antibody concentration via the addition of small volumes of solutions with increasing concentrations of target. Dissociation constants were obtained using classic two-state dose-response curves. The apparently bi-linear (sharper) response obtained for the anti-Dig antibody probe, which, if fit to a two-state binding equation, produced an apparent dissociation constant of 5 nM (FIGS. 2 and 3), thus suggesting that the true dissociation constant of this system was lower than the 4 nM probe concentration employed. Kinetic fluorescence data were obtained using Cary Eclipse Fluorimeter with excitation at 480 (± 5) nm and monitoring the total fluorescence above 517 nM.

Electrochemical measurements in buffer (1M NaCl, 0.05%) were performed at room temperature using a PalmSens potentiostat and a screen printed three-electrode cell containing a carbon-based ink counter electrode a silver-based ink reference electrode and a gold-based ink working electrode. Electrodes were fabricated as described in the literature using a high probe density. The set of probes with recognition elements were incubated for 5 minutes with the sample solution. After this incubation period, 100 uL of this solution was casted on the surface of the screen printed electrode and allowed to react for 10 minutes. Square wave voltammograms were collected at 60 Hz from -0.05 to -0.45 in increments of 0.001 V vs. silver-based reference with an amplitude of 50 mV. Peak currents were automatically taken using the PalmSens Instruments software.

## Claims

1. A nucleotide-based nanoswitch system for detecting one or more targets in a sample, the system comprising a set of two or more oligonucleotide probes configured to provide a target binding-induced structural change of one or more of the oligonucleotide probes by a proximity-based hybridization reaction triggered by an increase in local concentration of the two or more oligonucleotide probes, wherein, upon contact of said oligonucleotide probes with the one or more targets, hybridization between hybridization sequences of said probes occurs, thereby producing a detectable change in a signal from one of the probes when the one or more of the oligonucleotide probes are contacted by the one or more targets, wherein said one or more targets are protein analytes, in particular an antibody or a protein antigen, wherein said probes comprise:
a first probe containing a target binding moiety and a hybridization sequence;
a second probe containing a second target binding moiety and at least a hybridization sequence configured to form a duplex with the hybridization sequence of the first probe, said second probe further comprising a first signaling moiety and a second signaling moiety, wherein the first signaling moiety comprises an electrochemical reporter and the second signaling moiety comprises an electrode.

2. The nucleotide-based nanoswitch system of claim 1, wherein the probes comprise:
said first probe containing said target binding moiety and said hybridization sequence;
said second probe containing said second target binding moiety, two self-hybridization sequences and a hybridization sequence,
wherein the two self-hybridization sequences of the second probe have two self-complementary ends configured to create a stem-loop configuration and the hybridization sequence of the second probe is configured to be complementary to the hybridization sequence of the first probe to form a duplex with the hybridization sequence of the first probe, such that said duplex formation is inhibited in the absence of the target;
said first signaling moiety and said second signaling moiety of said second probe being configured such that the position of the first signaling moiety is in proximity of the second signaling moiety in absence of target and in the presence of the stem-loop configuration;
wherein upon binding of the one or more targets to both the first target binding moiety of the first probe and the second target binding moiety of the second probe, an increase in local concentration of the first probe and second probe is caused, the increase in local concentration of the first probe and second probe allowing the hybridization between the two hybridization sequences to form the duplex between the sequence of the first probe and the sequence of the second probe, causing thereby the opening of the stem-loop configuration thus changing the distance between the first signaling moiety and the second signaling moiety in the first probe and producing a detectable change in a signal from the first and second signaling moieties of the second probe.

3. The nucleotide-based nanoswitch system of claim 1, wherein the probes comprise:
said first probe containing said target binding moiety and said hybridization sequence;
said second probe containing said second target binding moiety and a first and a second hybridization sequences,
wherein the first and second hybridization sequences are configured to form a duplex with the hybridization sequence of the first probe;
a third probe containing a signaling moiety and a hybridization sequence configured to form a duplex with the first hybridization sequence of the second probe,
wherein the duplex formation between the hybridization sequence of the first probe and the first and second hybridization sequences of the second probe is inhibited in the absence of the target;
wherein upon binding of the one or more targets to both the first target binding moiety of the first probe and the second target binding moiety of the second probe an increase in local concentration of the first probe and second probe is caused, the increase in local concentration of the first probe and second probe allowing the formation of the duplex between the hybridization sequence of the first probe and the two hybridization sequences of the second probe to form the duplex between the sequence of the first probe and the sequence of the second probe causing thereby the release of the third probe and producing a detectable change in a signal from the first and second signaling moieties of the second probe.

4. The nucleotide-based nanoswitch system of claims 2 or 3, wherein the first target binding moiety and the second target binding moiety are bound directly to the two probes, further wherein the set of probes further comprises a third probe that contains a target binding moiety and a third hybridization sequence and wherein the second probe does not contain the target binding moiety but also contains an additional hybridization sequence that is configured to bound to the third hybridization sequence of the third probe.

5. The nucleotide-based nanoswitch system of claims 2 or 3, wherein the first probe does not contain the target binding moiety but also contains an additional hybridization sequence that is configured to bound to a fourth probe that contains a target binding moiety and a fourth hybridization sequence that is configured to bound to the additional hybridization sequence of the first probe.

6. The nucleotide-based nanoswitch system of any of claims 2 to 5, wherein the target is a protein antigen and the probes comprise linear probes and one or more hairpin probes, and wherein the target binding moieties are present on the linear probes and comprise molecules selected between antibodies, or single-chain variable fragments (Sc-Fv), affybodies, aptabodies, Fab (Fragment antigen binding, and variants F(ab')2, F(ab') and chemically linked F(ab')2)), single domain antibodies, said molecules specifically binding the target in different epitopes, wherein after hybridization between complementary sequences of hairpin probe and sequence of linear probe, an hairpin complex containing said molecules is formed, wherein when antigen is present in the sample, it is recognized by the moieties, local concentration of the probes increases so that the hybridization between one of the linear probe and hairpin complex generates the signal.

7. The nucleotide-based nanoswitch system of any of claims 2 to 5, wherein the probes comprise one hairpin probe and one linear probe which is biotinylated or coupled to magnetic nanoparticle, and is partially constituted by a RNA or PNA strand, which is complementary to the stem loop of the hairpin probe, wherein the target binding induces an increase of the local concentration of the probes and consequently the formation of hybrid duplex, DNA-RNA or DNA-PNA, an antibody, or another molecule recognizing specifically the hybrid duplex, conjugated with an enzyme, binds the duplex formation only when the target is present, wherein the enzyme is able to start an enzymatic reaction measurable in terms of chemiluminescence, absorbance or through amperometry.

8. The nucleotide-based nanoswitch system of any of claims 2 to 5, wherein a nucleic acid intercalating dye is added to the probes, the dye binding DNA only when it is double-stranded and thereby emitting a fluorescent signal.

9. A method of detecting a target in a sample by a proximity-based hybridization reaction, the method comprising:
contacting a set of oligonucleotide probes according to any of claims 1 to 8 with the sample, whereby the target selectively binds to both the first target binding moiety and the second target binding moiety to form a complex with the set of probes of any of claims 1 to 8 and the target, wherein, upon contact of said oligonucleotide probes with the target, hybridization between hybridization sequences of said probes occurs; and detecting the presence or absence of the complex with the set of probes of any of claims 1 to 8 and the target,
wherein said is a protein analyte, in particular an antibody or a protein antigen.

10. A method of detecting a second target in a sample by a proximity-based hybridization reaction, the method comprising:
contacting a set of oligonucleotide probes of any of claims 1 to 8 with the sample, whereby the target selectively binds to both the first target binding sequence and the second target binding sequence to form a complex with the set of probes of any of claims 1 to 8, wherein, upon contact of said oligonucleotide probes with the targets, hybridization between hybridization sequences of said probes occurs; contacting the first target with a second target, whereby the second target selectively binds the target and inhibits formation of the complex with the set of probes of any of claims 1 to 8 and the target; and detecting the presence or absence of the complex with the set of probes of any of claims 1 to 8 and the target,
wherein said one or more targets are protein analytes, in particular an antibody or a protein antigen.

## Patentansprüche

1. Nukleotidbasiertes Nano-Switch-System zur Detektion eines oder mehrerer Targets in einer Probe, wobei das System ein Set von zwei oder mehr Oligonukleotid-Sonden aufweist, die eingerichtet sind eine target-bindungsinduzierte Strukturänderung von einer oder mehreren der Oligonukleotid-Sonden hervorzurufen, durch eine auf Nähe basierende Hybridisierungsreaktion, ausgelöst durch einen Anstieg der lokalen Konzentration der zwei oder mehr Oligonukleotid-Sonden, wobei bei Kontakt der Oligonukleotid-Sonden mit dem einen oder den mehreren Targets eine Hybridisierung zwischen den Hybridisierungssequenzen der Sonden auftritt, wodurch eine detektierbare Änderung eines Signals von einer der Sonden erzeugt wird, wenn die eine oder die mehreren der Oligonukleotid-Sonden von dem einen oder den mehreren der Targets kontaktiert werden, wobei das eine oder die mehreren Targets Protein-Analyten sind, insbesondere Antikörper oder Protein-Antigene, wobei die Sonden aufweisen:
eine erste Sonde enthaltend einen Target-Bindungsteil und eine Hybridisierungssequenz;
eine zweite Sonde enthaltend einen zweiten Target-Bindungsteil und mindestens eine Hybridisierungssequenz, die eingerichtet ist ein Duplex mit der Hybridisierungssequenz der ersten Sonde zu bilden, wobei die zweite Sonde ferner einen ersten Signalgebungsteil und einen zweiten Signalgebungsteil aufweist, wobei der erste Signalgebungsteil einen elektrochemischen Reporter aufweist und der zweite Signalgebungsteil eine Elektrode aufweist.

2. Nukleotidbasiertes Nano-Switch-System nach Anspruch 1, wobei die Sonden aufweisen:
die erste Sonde, enthaltend den Target-Bindungsteil und die Hybridisierungssequenz;
die zweite Sonde, enthaltend den zweiten Target-Bindungsteil, zwei Selbst-Hybridisierungssequenzen und eine Hybridisierungssequenz,
wobei die beiden Selbst-Hybridisierungssequenzen der zweiten Sonde zwei Selbst-Komplementär-Enden aufweisen, die eingerichtet sind eine Stamm-Schleife-Konfiguration zu bilden und die Hybridisierungssequenz der zweiten Sonde eingerichtet ist komplementär zu der Hybridisierungssequenz der ersten Sonde zu sein, um ein Duplex mit der Hybridisierungssequenz der ersten Sonde so zu bilden, dass die Duplexbildung in Abwesenheit des Targets gehemmt ist;
wobei der erste Signalgebungsteil und der zweite Signalgebungsteil der zweiten Sonde so eingerichtet sind, dass in Abwesenheit des Targets und dem Vorliegen der Stamm-Schleife-Konfiguration die Position des ersten Signalgebungsteils in der Nähe des zweiten Signalgebungsteils liegt;
wobei bei Bindung des einen oder der mehreren Targets an sowohl den ersten Target-Bindungsteil der ersten Sonde als auch den zweiten Target-Bindungsteil der zweiten Sonde ein Anstieg der lokalen Konzentration der ersten Sonde und der zweiten Sonde verursacht wird, die Erhöhung der lokalen Konzentration der ersten Sonde und der zweiten Sonde die Hybridisierung zwischen den beiden Hybridisierungssequenzen ermöglicht um den Duplex zwischen der Sequenz der ersten Sonde und der Sequenz der zweiten Sonde zu bilden, wodurch die Öffnung der Stamm-Schleife-Konfiguration verursacht wird und dadurch der Abstand zwischen dem ersten Signalgebungsteil und dem zweiten Signalgebungsteil in der ersten Sonde geändert wird und eine detektierbare Änderung in einem Signal von den ersten und zweiten Signalgebungsteilen der zweiten Sonde erzeugt wird.

3. Nukleotidbasiertes Nano-Switch-System nach Anspruch 1, wobei die Sonden aufweisen:
die erste Sonde, enthaltend den Target-Bindungsteil und die Hybridisierungssequenz;
die zweite Sonde, enthaltend den zweiten Target-Bindungsteil und eine erste und eine zweite Hybridisierungssequenz, wobei die erste und zweite Hybridisierungssequenzen eingerichtet sind ein Duplex mit der Hybridisierungssequenz der ersten Sonde zu bilden;
eine dritte Sonde, enthaltend einen Signalgebungsteil und eine Hybridisierungssequenz, die eingerichtet ist, ein Duplex mit der ersten Hybridisierungssequenz der zweiten Sonde zu bilden,
wobei die Duplexbildung zwischen der Hybridisierungssequenz der ersten Sonde und der ersten und zweiten Hybridisierungssequenzen der zweiten Sonde in Abwesenheit des Targets gehemmt ist;
wobei bei Bindung des einen oder der mehreren Targets an sowohl den ersten Target-Bindungsteil der ersten Sonde als auch den zweiten Target-Bindungsteil der zweiten Sonde ein Anstieg der lokalen Konzentration der ersten Sonde und der zweiten Sonde bewirkt wird, die Erhöhung der lokalen Konzentration der ersten Sonde und der zweiten Sonde die Bildung des Duplex zwischen der Hybridisierungssequenz der ersten Sonde und den beiden Hybridisierungssequenzen der zweiten Sonde ermöglicht, um den Duplex zwischen der Sequenz der ersten Sonde und der Sequenz der zweiten Sonde zu bilden, wodurch die Freisetzung der dritten Sonde bewirkt wird und eine detektierbare Änderung in einem Signals der ersten und zweiten Signalgebungsteilen der zweiten Sonde erreicht wird.

4. Nukleotidbasiertes Nano-Switch-System nach Anspruch 2 oder 3, wobei der erste Target-Bindungsteil und der zweite Target-Bindungsteil direkt an die beiden Sonden gebunden sind, wobei ferner das Set von Sonden ferner eine dritte Sonde aufweist, die einen Target-Bindungsteil und eine dritte Hybridisierungssequenz enthält und wobei die zweite Sonde nicht den Target-Bindungsteil enthält, aber auch eine zusätzliche Hybridisierungssequenz, die eingerichtet ist an die dritte Hybridisierungssequenz der dritten Sonde zu binden.

5. Nukleotidbasiertes Nano-Switch-System nach Anspruch 2 oder 3, wobei die erste Sonde nicht den Target-Bindungsteil enthält, aber auch eine zusätzliche Hybridisierungssequenz, die eingerichtet ist an eine vierte Sonde zu binden, die einen Target-Bindungsteil und eine vierte Hybridisierungssequenz enthält, die eingerichtet ist an die zusätzliche Hybridisierungssequenz der ersten Sonde zu binden.

6. Nukleotidbasiertes Nano-Switch-System nach irgendeinem der Ansprüche 2 bis 5, wobei das Target ein Protein-Antigen ist und die Sonden Linear-Sonden und eine oder mehrere Haarnadel-Sonden aufweisen, und wobei die Target-Bindungsteile auf den Linear-Sonden vorliegen und Moleküle aufweisen, ausgewählt aus Antikörpern oder einkettigen variablen Fragmenten (Sc-Fv), Affybodies, Aptabodies, Fab (Fragment-Antigen-Bindung und Varianten F(ab')2, F(ab') und chemisch gebundenes F(ab')2)), Einzeldomänenantikörper, wobei die Moleküle spezifisch das Target in verschiedenen Epitopen binden, wobei nach Hybridisierung zwischen komplementären Sequenzen der Haarnadelsonde und der Sequenz der Linear-Sonde ein Haarnadelkomplex gebildet wird, der die Moleküle enthält, wobei, wenn Antigen in der Probe vorhanden ist, dies von den Teilen erkannt wird, die lokale Konzentration der Sonden so zunimmt, dass die Hybridisierung zwischen einer der Linear-Sonden und dem Haarnadelkomplex das Signal erzeugt.

7. Nukleotidbasiertes Nano-Switch-System nach irgendeinem der Ansprüche 2 bis 5, wobei die Sonden eine Haarnadel-Sonde und eine Linear-Sonde, welche biotinyliert oder an magnetische Nanopartikel gekoppelt ist, aufweisen und teilweise von einem RNA- oder PNA-Strang gebildet werden, welcher komplementär zu der Stamm-Schleife der Haarnadel-Sonde ist, wobei die Target-Bindung eine Erhöhung der lokalen Konzentration der Sonden verursacht und folglich die Bildung von einem Hybrid-Duplex, DNA-RNA oder DNA-PNA, ein Antikörper oder ein anderes Molekül das spezifisch den Hybrid-Duplex erkennt, konjugiert mit einem Enzym, bindet die Duplexstruktur nur dann, wenn das Target vorhanden ist, wobei das Enzym in der Lage ist eine enzymatische Reaktion in Gang zu setzen, die durch Chemilumineszenz, Absorption oder Amperometrie messbar ist.

8. Nukleotidbasiertes Nano-Switch-System nach irgendeinem der Ansprüche 2 bis 5, wobei den Sonden ein Nukleinsäure-Interkalierend-Farbstoff zugesetzt ist, wobei der Farbstoff DNA nur dann bindet, wenn sie doppelsträngig ist und dadurch ein Fluoreszenzsignal emittiert.

9. Verfahren zur Detektion eines Targets in einer Probe durch eine auf Nähe basierende Hybridisierungsreaktion, das Verfahren aufweisend:
Kontaktieren eines Sets von Oligonukleotid-Sonden nach irgendeinem der Ansprüche 1 bis 8 mit der Probe, wobei das Target selektiv an sowohl den ersten Target-Bindungsteil als auch den zweiten Target-Bindungsteil bindet, um einen Komplex mit dem Set von Sonden nach irgendeinem der Ansprüche 1 bis 8 und dem Target zu bilden, wobei bei Kontakt der Oligonucleotid-Sonden mit dem Target eine Hybridisierung zwischen den Hybridisierungssequenzen der Sonden auftritt; und
Nachweisen der Anwesenheit oder Abwesenheit des Komplexes mit dem Set von Sonden nach irgendeinem der Ansprüche 1 bis 8 und dem Target,
wobei es sich bei diesem um einen Protein-Analyten, insbesondere einen Antikörper oder ein Protein-Antigen handelt.

10. Verfahren zur Detektion eines zweiten Targets in einer Probe durch eine auf Nähe basierende Hybridisierungsreaktion, das Verfahren aufweisend:
Kontaktieren eines Sets von Oligonukleotid-Sonden nach irgendeinem der Ansprüche 1 bis 8 mit der Probe, wobei das Target selektiv an sowohl die erste Target-Bindesequenz als auch die zweite Target-Bindesequenz bindet, um einen Komplex mit dem Set von Sonden nach irgendeinem der Ansprüche 1 bis 8 zu bilden, wobei bei Kontakt der Oligonukleotid-Sonden mit den Targets Hybridisierung zwischen Hybridisierungssequenzen der Sonden auftritt;
Kontaktieren des ersten Targets mit einem zweiten Target, wobei das zweite Target selektiv das Target bindet und die Bildung des Komplexes mit dem Set von Sonden nach irgendeinem der Ansprüche 1 bis 8 und dem Target hemmt;
und Detektieren der Anwesenheit oder Abwesenheit des Komplexes mit dem Set von Sonden nach irgendeinem der Ansprüche 1 bis 8 und dem Target,
wobei das eine oder die mehreren Targets Protein-Analyten sind, insbesondere Antikörper oder Protein-Antigene.

## Revendications

1. Système de nanocommutateur à base de nucléotides pour détecter une ou plusieurs cibles dans un échantillon, le système comprenant un ensemble de deux sondes oligonucléotidiques ou plus configurées pour fournir un changement structurel induit par une liaison de cible d'une ou plusieurs des sondes oligonucléotidiques par une réaction d'hybridation basée sur la proximité déclenchée par une augmentation de concentration locale des deux sondes oligonucléotidiques ou plus, dans lequel, lors d'un contact desdites sondes oligonucléotidiques avec les une ou plusieurs cibles, une hybridation entre des séquences d'hybridation desdites sondes survient, en produisant ainsi un changement détectable dans un signal provenant de l'une des sondes lorsque les une ou plusieurs sondes oligonucléotidiques sont mises en contact par les une ou plusieurs cibles, dans lequel lesdites une ou plusieurs cibles sont des analytes protéiques, en particulier un anticorps ou un antigène protéique, dans lequel lesdites sondes comprennent :
une première sonde contenant un fragment de liaison de cible et une séquence d'hybridation ;
une deuxième sonde contenant un second fragment de liaison de cible et au moins une séquence d'hybridation configurée pour former un duplex avec la séquence d'hybridation de la première sonde, ladite deuxième sonde comprenant en outre un premier fragment de signalisation et un second fragment de signalisation, dans lequel le premier fragment de signalisation comprend un reporteur électrochimique et le second fragment de signalisation comprend une électrode.

2. Système de nanocommutateur à base de nucléotides selon la revendication 1, dans lequel les sondes comprennent :
ladite première sonde contenant ledit fragment de liaison de cible et ladite séquence d'hybridation ;
ladite deuxième sonde contenant ledit second fragment de liaison de cible, deux séquences d'auto-hybridation et une séquence d'hybridation,
dans lequel les deux séquences d'auto-hybridation de la deuxième sonde ont deux extrémités auto-complémentaires configurées pour créer une configuration en tige-boucle et la séquence d'hybridation de la deuxième sonde est configurée pour être complémentaire de la séquence d'hybridation de la première sonde pour former un duplex avec la séquence d'hybridation de la première sonde, de telle sorte que ladite formation de duplex est inhibée en l'absence de la cible ;
ledit premier fragment de signalisation et ledit second fragment de signalisation de ladite deuxième sonde étant configurés de telle sorte que la position du premier fragment de signalisation soit à proximité du second fragment de signalisation en l'absence de cible et en présence de la configuration tige-boucle ;
dans lequel lors de la liaison des une ou plusieurs cibles à la fois au premier fragment de liaison de cible de la première sonde et au second fragment de liaison de cible de la deuxième sonde, une augmentation de la concentration locale de la première sonde et de la deuxième sonde est provoquée, l'augmentation de la concentration locale de la première sonde et de la deuxième sonde permettant l'hybridation entre les deux séquences d'hybridation pour former le duplex entre la séquence de la première sonde et la séquence de la deuxième sonde, en provoquant ainsi l'ouverture de la configuration tige-boucle et en changeant ainsi la distance entre le premier fragment de signalisation et le second fragment de signalisation dans la première sonde et en produisant un changement détectable d'un signal provenant des premier et second fragments de signalisation de la deuxième sonde.

3. Système de nanocommutateur à base de nucléotides selon la revendication 1, dans lequel les sondes comprennent :
ladite première sonde contenant ledit fragment de liaison de cible et ladite séquence d'hybridation ;
ladite deuxième sonde contenant ledit second fragment de liaison de cible et des première et seconde séquences d'hybridation,
dans lequel les première et seconde séquences d'hybridation sont configurées pour former un duplex avec la séquence d'hybridation de la première sonde ;
une troisième sonde contenant un fragment de signalisation et une séquence d'hybridation configurée pour former un duplex avec la première séquence d'hybridation de la deuxième sonde,
dans lequel la formation de duplex entre la séquence d'hybridation de la première sonde et les première et seconde séquences d'hybridation de la deuxième sonde est inhibée en l'absence de la cible ;
dans lequel lors d'une liaison des une ou plusieurs cibles à la fois au premier fragment de liaison de cible de la première sonde et au second fragment de liaison de cible de la deuxième sonde, une augmentation de la concentration locale de la première sonde et de la deuxième sonde est provoquée, l'augmentation de la concentration locale de la première sonde et de la deuxième sonde permettant la formation du duplex entre la séquence d'hybridation de la première sonde et les deux séquences d'hybridation de la deuxième sonde pour former le duplex entre la séquence de la première sonde et la séquence de la deuxième sonde, en provoquant ainsi la libération de la troisième sonde et en produisant un changement détectable dans un signal provenant des premier et second fragments de signalisation de la deuxième sonde.

4. Système de nanocommutateur à base de nucléotides selon les revendications 2 ou 3, dans lequel le premier fragment de liaison de cible et le second fragment de liaison de cible sont liés directement aux deux sondes, en outre dans lequel l'ensemble de sondes comprend en outre une troisième sonde qui contient un fragment de liaison de cible et une troisième séquence d'hybridation et dans lequel la deuxième sonde ne contient pas le fragment de liaison de cible mais contient également une séquence d'hybridation supplémentaire qui est configurée pour se lier à la troisième séquence d'hybridation de la troisième sonde.

5. Système de nanocommutateur à base de nucléotides selon la revendication 2 ou 3, dans lequel la première sonde ne contient pas le fragment de liaison de cible mais contient également une séquence d'hybridation supplémentaire qui est configurée pour se lier à une quatrième sonde qui contient un fragment de liaison de cible et une quatrième séquence d'hybridation qui est configurée pour se lier à la séquence d'hybridation supplémentaire de la première sonde.

6. Système de nanocommutateur à base de nucléotides selon l'une quelconque des revendications 2 à 5, dans lequel la cible est un antigène protéique et les sondes comprennent des sondes linéaires et une ou plusieurs sondes en épingle à cheveux, et dans lequel les fragments de liaison de cible sont présents sur les sondes linéaires et comprennent des molécules sélectionnées entre des anticorps, ou des fragments variables à chaîne unique (Sc-Fv), des affibodies, des aptabodies, Fab (Fragment de liaison d'antigène et variants F(ab')2, F(ab') et F(ab')2) lié chimiquement)), anticorps à domaine unique, lesdites molécules se liant spécifiquement à la cible dans différents épitopes, dans lequel après hybridation entre des séquences complémentaires de sonde en épingle à cheveux et une séquence de sonde linéaire, un complexe en épingle à cheveux contenant lesdites molécules est formé, dans lequel, lorsque l'antigène est présent dans l'échantillon, il est reconnu par les fragments, la concentration locale des sondes augmente de sorte que l'hybridation entre l'un de la sonde linéaire et du complexe en épingle à cheveux génère le signal.

7. Système de nanocommutateur à base de nucléotides selon l'une quelconque des revendications 2 à 5, dans lequel les sondes comprennent une sonde en épingle à cheveux et une sonde linéaire qui est biotinylée ou couplée à une nanoparticule magnétique, et est partiellement constituée par un brin d'ARN ou d'APN, qui est complémentaire. à la tige-boucle de la sonde en épingle à cheveux, dans lequel la liaison de cible induit une augmentation de la concentration locale des sondes et par conséquent la formation d'un duplex hybride, ADN-ARN ou ADN-APN, un anticorps, ou une autre molécule reconnaissant spécifiquement le duplex hybride, conjugué à une enzyme, se lie à la formation de duplex uniquement lorsque la cible est présente, dans lequel l'enzyme est capable de démarrer une réaction enzymatique mesurable en termes de chimioluminescence, d'absorbance ou par ampérométrie.

8. Système de nanocommutateur à base de nucléotides selon l'une quelconque des revendications 2 à 5, dans lequel un colorant d'intercalation d'acide nucléique est ajouté aux sondes, le colorant ne liant l'ADN que lorsqu'il est double brin et émettant ainsi un signal fluorescent.

9. Procédé de détection d'une cible dans un échantillon par une réaction d'hybridation basée sur la proximité, le procédé comprenant les étapes consistant à :
mettre en contact un ensemble de sondes oligonucléotidiques selon l'une quelconque des revendications 1 à 8 avec l'échantillon, moyennant quoi la cible se lie sélectivement à la fois au premier fragment de liaison de cible et au second fragment de liaison de cible pour former un complexe avec l'ensemble de sondes de l'une quelconque des revendications 1 à 8 et la cible, dans lequel, lors du contact desdites sondes oligonucléotidiques avec la cible, une hybridation entre des séquences d'hybridation desdites sondes survient ; et détecter la présence ou l'absence du complexe avec l'ensemble de sondes selon l'une quelconque des revendications 1 à 8 et la cible,
dans lequel ledit est un analyte protéique, en particulier un anticorps ou un antigène protéique.

10. Procédé de détection d'une seconde cible dans un échantillon par une réaction d'hybridation basée sur la proximité, le procédé comprenant les étapes consistant à :
mettre en contact un ensemble de sondes oligonucléotidiques selon l'une quelconque des revendications 1 à 8 avec l'échantillon, moyennant quoi la cible se lie sélectivement à la fois à la première séquence de liaison cible et à la seconde séquence de liaison cible pour former un complexe avec l'ensemble de sondes de l'une quelconque des revendications 1 à 8, dans lequel, lors d'un contact desdites sondes oligonucléotidiques avec les cibles, une hybridation entre des séquences d'hybridation desdites sondes survient ; mettre en contact la première cible avec une seconde cible, moyennant quoi la seconde cible se lie sélectivement à la cible et inhibe la formation du complexe avec l'ensemble de sondes de l'une quelconque des revendications 1 à 8 et la cible ; et détecter la présence ou l'absence du complexe avec l'ensemble de sondes de l'une quelconque des revendications 1 à 8 et la cible,
dans lequel lesdites une ou plusieurs cibles sont des analytes protéiques, en particulier un anticorps ou un antigène protéique.


### SEQUENCE LISTING

<110> ULISSE BIOMED S.r.l.
<120> NUCLEOTIDE-BASED NANOSWITCH AND METHODS FOR THE DETECTION OF
   ANTIBODIES AND OTHER ANALYTES
<130> SEQUENCE_LISTING
<160> 12
<170> BiSSAP 1.3.6
<210> 1
   <211> 27
   <212> DNA
   <213> Artificial Sequence
<220>
   <223> Portion of probe
<400> 1
   taggcgttct agatatgcaa tcgcctt 27
<210> 2
   <211> 15
   <212> DNA
   <213> Artificial Sequence
<220>
   <223> Portion of probe
<400> 2
   gtctcagaat gtaag 15
<210> 3
   <211> 20
   <212> DNA
   <213> Artificial Sequence
<220>
   <223> Portion of probe
<400> 3
   tttttcttac attctgagac 20
<210> 4
   <211> 30
   <212> DNA
   <213> Artificial Sequence
<220>
   <223> Portion of probe
<400> 4
   tttttttttt tttttattgc atatctagaa 30
<210> 5
   <211> 27
   <212> DNA
   <213> Artificial Sequence
<220>
   <223> Portion of probe
<400> 5
   tgagcggtat agcctaattc gcgctct 27
<210> 6
   <211> 15
   <212> DNA
   <213> Artificial Sequence
<220>
   <223> Portion of probe
<400> 6
   ctgtcacttt ctgag 15
<210> 7
   <211> 20
   <212> DNA
   <213> Artificial Sequence
<220>
   <223> Portion of probe
<400> 7
   tttttctcag aaagtgacag 20
<210> 8
   <211> 30
   <212> DNA
   <213> Artificial Sequence
<220>
   <223> Portion of probe
<400> 8
   tttttttttt tttttcgaat taggctatac 30
<210> 9
   <211> 32
   <212> DNA
   <213> Artificial Sequence
<220>
   <223> Portion of probe
<400> 9
   tctcaagatc ctaggtaagt cgtttttttt tt 32
<210> 10
   <211> 19
   <212> DNA
   <213> Artificial Sequence
<220>
   <223> Portion of probe
<400> 10
   cttacctagg atcttgaga 19
<210> 11
   <211> 32
   <212> DNA
   <213> Artificial Sequence
<220>
   <223> Portion of probe
<400> 11
   cgacttacct aggatctaat tttttttttt tt 32
<210> 12
   <211> 15
   <212> DNA
   <213> Artificial Sequence
<220>
   <223> Portion of probe
<400> 12
   tctcaagatc ctagg 15